(19) Europäisches Patentamt
European Patent Office
Office européen des brevets

(11) Publication number: **0 124 124 A2**

(12) # EUROPEAN PATENT APPLICATION

(21) Application number: 84104848.1

(22) Date of filing: 30.04.84

(51) Int. Cl.³: G 01 N 33/54

(30) Priority: 02.05.83 US 490712
26.01.84 US 574632

(43) Date of publication of application: 07.11.84 Bulletin 84/45

(84) Designated Contracting States: AT BE CH DE FR GB IT LI LU NL SE

(71) Applicant: ENZO BIOCHEM, INC., 325 Hudson Street, New York, N.Y. 10013 (US)

(72) Inventor: Yang, Huey-Lang, 76 Engle Street, Tenafly New Jersey (US)
Inventor: Kirtikar, Dollie, 42-72 80 Street, Elmhurst New York (US)
Inventor: Pergolizzi, Robert G., 375 New Bridge Road, New Milford New Jersey (US)

(74) Representative: Vossius Vossius Tauchner Heunemann Rauh, Siebertstrasse 4 P.O. Box 86 07 67, D-8000 München 86 (DE)

(54) **Method and materials useful for the analysis and detection of genetic material.**

(57) In techniques for the analysis of genetic material, such as DNA and/or RNA, wherein the genetic material is denatured, fixed to a substrate and hybridized with a probe, superior results are obtainable when there is employed as the probe a chemically labeled probe which, preferably after hybridization, has added or attached thereto, such as to the chemical label or moiety of the probe, an enzyme component effective upon contact with a chromogen to produce an insoluble color precipitate or product. Techniques for the analysis of genetic material which are improved in sensitivity, definition, accuracy and/or speed, by employing a chemically labeled probe, include such well known techniques as «Southern» blot analysis, «Northern» blot analysis, «Western» blot analysis, colony hybridization, plaque lifts, cytoplasmic dot hybridization, in situ hybridization to cells and/or tissues and other such techniques.

EP 0 124 124 A2

Our ref.: S 924 EP
Case: 20692
Enzo Biochem



April 30, 1984

0124124

# METHOD AND MATERIALS USEFUL FOR THE ANALYSIS AND DETECTION OF GENETIC MATERIAL

Techniques have been developed for the analysis of genetic material, such as DNA and/or RNA. For example, techniques have been developed for the mapping of genes, such as genes within cellular DNA fragments which have been separated by electrophoresis after fragmentation or splitting of the cellular DNA or genetic material by restriction endonucleases and other means and fixed to a substrate. Such techniques for the identification or analysis of the genetic material have employed a radioactive probe, such as a DNA probe containing radioactive phosphorous as a component of one or more of the nucleotides making up the probe. The probe would be applied to the prepared genetic material under conditions such that hybridization of the probe with the genetic material of interest occurs. Thereupon, the resulting formed hybrids containing the radioactive phosphorous labeled DNA probe hybridized with the DNA genetic material or fragment of interest would be detected by conventional means for the detection of radioactive material. The use of radioactive probes for the analysis of genetic material, such as DNA and/or RNA, is inconvenient for a number of reasons. For example, caution must be exercised in the handling of radioactive materials. The radioactive materials commonly employed, such as tritium or radioactive phosphorous have a relatively short half-life and must frequently be replenished. As a result of the above, the use of a radioactive probe in the analysis of genetic material also tends to be time-consuming since, because of the amount of intensity of the radioactivity to be detected, a prolonged exposure of radiation sensitive film is required, such as a number of days. Also, the definition of the genetic material containing

the hybridized radioactive probe often times tends to be fuzzy and not well defined.

It is an object of this invention to provide an improved technique for analysis of genetic materials and related materials wherein DNA and/or RNA material, after suitable treatment, is hybridized with a non-radioactive probe.

It is another object of this invention to provide an improved technique for the analysis of genetic and other materials employing a probe.

It is yet another object of this invention to provide an improved technique for the analysis of genetic material, such as DNA or RNA fragments, employing a probe capable of hybridizing with the genetic material to be identified or located and wherein the presence of the resulting probe, now hybridized with the material to be identified, is readily elicited and its presence defined.

Yet another object of this invention is to provide a technique to confirm the presence of genetic material when the analysis of the genetic material yields a negative result.

How these and other objects of this invention are achieved will become apparent in the light of the accompanying disclosure.

0124124

Techniques for the analysis and/or identification of various materials, such as proteins or genetic materials such as DNA and/or RNA fragments or gene-containing DNA fragments or genes, are improved by employing a chemically-labeled probe. In techniques in accordance with this invention for the analysis of such material employing a chemically-labeled probe, the material is denatured and fixed, such as fixed to a substrate or fixed in situ, and then hybridized with a chemically-labeled probe. The resulting hybrid containing the chemically-labeled probe has added or attached thereto, such as the chemical label or moiety of the probe, a chemical component, such as an enzyme component, effective upon contact with a chromogen or similar material to produce with a chromogen or similar material a colored or visible product or insoluble color precipitate which is usually quickly readily observable by visual inspection, especially in contrast to the use of a radioactive labeled probe which requires many hours and special equipment, e.g., film, before the presence or absence of the hybrid containing the radioactive probe can be identified or located. The techniques which are improved by employing therein a chemically-labeled probe in accordance with the practices of this invention are many and varied. For example, the technique for the analysis of genetic material, such as DNA fragments, known as Southern blot analysis is particularly applicable in the practices of this invention. The techniques for effecting Southern blot analysis of genetic material, such as DNA fragments, is described in Journal of Molecular Biology (1975) 98, 503-517, in the entitled "Detection of Specific Sequences Among DNA Fragments Separated by

Gel Electrophoresis." Another technique for genetic analysis which is improved by employing a chemically-labeled probe in accordance with this invention is the so-called colony hybridization. The colony hybridization technique for genetic analysis is described in Methods of Enzymology, Edited by Ray Wu (1979), Vol. 68, pp. 379-409, published by Academic Press, see particularly the article by M. Grunstein and J. Wallis entitled "Colony Hybridization" and in the Proceedings of the National Academy of Sciences, U.S.A., Vol. 72, No. 10, pp. 3961-3965, October, 1975 in the article by M. Grunstein and D.S. Hogness entitled "Colony Hybridization: A Method for the Isolation of Cloned DNAs that Contain a Specific Gene." Another technique for genetic analysis, which is improved by employing a chemical label probe in accordance with the practices of this invention is called cytoplasmic dot hybridization, see the article by B.A. White and F.C. Brancroft entitled "Simple Analysis of Relative mRNA Levels in Multiple Small Cell or Tissue Samples", published in The Journal of Biological Chemistry, (1982), Vol. 257, No. 15, pp 8569-8572. Another related technique for the analysis of genetic material is described in the Proceedings of the National Academy of Sciences, U.S.A., (1977), Vol. 74, No. 12, pp. 5350-5354, the article by J.C. Alwine, D.J. Kemp and G.R. Stark entitled "Method for Detection of Specific RNAs in agarose gels by transfer to diazobenzyloxymethyl-Paper and Hybridization with DNA Probes". Other techniques and applications of genetic analysis which are improved by employing chemically-labeled probes in accordance with this invention are described in Gene, (1980) 10, pp. 63-67, the article by D. Hanahan and M. Meselson entitled "Plasmid Screening at High Colony Density". Other techniques and applications involving the analysis of

genetic materials which are improved by employing a chemically-labeled probe in accordance with the practices of this invention are set forth in the article by J.C. Chang et. al., entitled "A Sensitive New Prenatal Test for Sickle-Cell Anemia", which appeared in The New England Journal of Medicine, July 1, 1982, pp. 30-36; and the article by J.T. Wilson et. al., entitled "Use of Restriction Endonucleases for Mapping the Allele for Beta$^S$-Globin", which appeared in the Proceedings of the National Academy of Sciences, U.S.A., Vol. 79, pp. 3628-3631, June 1982, and the article by B.J. Conner et. al., entitled "Detection of Sickle Cell Beta$^S$-Globin Allele by Hybridization with Synthesis Oligonucleotides" in the Proceedings of the National Academy of Sciences, U.S.A., Vol. 80, pp. 278-282, January, 1983.

Another technique which can be improved by employing therein a chemically labeled probe in accordance with the practices of this invention is that the genetic material can be fixed *in situ* such as *in situ* hybridization to cells and/or tissues. *In situ* hybridization to cells and/or tissues permits one to analyze the genetic material without purifying such material. This results in a rapid analysis of the genetic material. This is particularly advantageous in a clinical environment where it is desirable to analyze the genetic material rapidly. Also, *in situ* hybridization to cell and/or tissues permits one detect location of the target genetic material within the cells or tissues. Techniques for *in situ* hybridization to cells and/or tissues are described in the article by Pardue entitled "Nucleic Acid Hybridization to the DNA of Cytological Preparations", which appeared in Methods in Cell Biology (1975) pp. 1-16, and in the article by Brahic et. al., entitled "Detection of Viral Sequences of Low Reiteration Frequency by *In Situ* Hybridization"

in the Proc. Natl. Acad. Sci. U.S.A., Vol. 75, pp. 6125-6129, December 1978 and in the article by Gerhard et. al., entitled "Localization of a Unique Gene by Direct Hybridization In Situ" in the Proc. Natl. Acad. Sci. U.S.A., Vol. 78, pp. 3755-3759, June 1981.

In general, those techniques of genetic analysis involving the denaturing of genetic material, such as DNA fragments, followed by fractionation or separation of the genetic material of interest, such as by electrophoresis and fixing the thus-treated genetic material to a suitable substrate, such as nitrocellulose paper, followed by hybridization are improved by employing as the hybridizing probe, a chemically labeled probe, by way of identification, such techniques, as indicated hereinabove, have been referred to as dot blots, colony hybridization, plaque lifts, Southern, Northern and Western blots. The disclosures of all the above-identified publications, particularly with respect to the various techniques involved, substantially all of which are applicable in the practices of this invention, are herein incorporated and made part of this disclosure.

The techniques for the analysis of genetic material within the practices of the invention can be used to analyze essentially any genetic material. For example, viruses, bacteria and genetic defects can be analyzed. Nonlimiting examples of viruses and bacteria are Herpes Simplex Viruses I and II, Hepatitis B, Cytomegalovirus, Epstein Barr Virus, Hepatitis A, Neisseira gonorrhoeae, Mycoplasma, Streptococcus A, Chlamydia, Ampicillin resistance, Syphillis, E. coli, Mycobacterium, Meningococci, Haemophilis, Enterovirus, Staphtlococcus, Proteus, Klebsillia aerogenes, Klebsillia pneumonia, Pseudomonas, Papilloma virus, pneumococcus, RSV,

Parainfluenza, Legionella, Camphylobacter, Salmonella, Shigella, Rotavirus and Adenovirus. Nonlimiting examples of genetic defects that can be analyzed are cystic fibrosis, Huntington chorea, certain forms of muscular dystrophies, sickle cell anemia, Hemophila and a variety of cancers and other metabolic disorders.

Various types of chemically-labeled probes, e.g., chemically-labeled polynucleotides, can be employed in the practices of this invention. For example, a particularly useful chemically-labeled probe is a probe which contains one or more biotin-labeled nucleotides, such as the biotinylated nucleotide 2'-deoxyuridine triphosphate 5-allylamine-biotin. Biotin-labeled polynucleotides useful as chemically-labeled probes in accordance with the practices of this invention are described in the Proceedings of the National Academy of Sciences, U.S.A., Vol. 78, No. 11, pp. 6633-6637, November 1981, in the article by P.R. Langer et. al., entitled "Enzymatic Synthesis of Biotin-Labeled Polynucleotides: Novel Nucleic Acid Affinity Probes". Chemically-labeled probes are also described in EP-A- 63 879 and in EP-A-97 373. The disclosures of the above-identified article and the above-identified European patent applications are herein incorporated and made part of this disclosure.

With respect to the chemically-labeled probes which are useful in the practices of this invention, compounds or nucleotides which have been prepared for incorporation into DNA, such as double-stranded DNA, and which are

also useful for the preparation of chemically-labeled probes in the practices of this invention, have the structure:

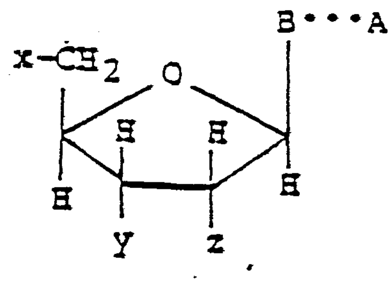

wherein B represents a purine, deazapurine, or pyrimidine moiety covalently bonded to the $C^{1'}$-position of the sugar moiety, provided that when B is purine or 7-deazapurine, it is attached at the $N^9$-position of the purine or deazapurine, and when B is pyrimidine, it is attached at the $N^1$-position;

wherein A represents a moiety consisting of at least three carbon atoms which is capable of forming a detectable complex with a polypeptide when the compound is incorporated into a double-stranded ribonucleic acid, deoxyribonucleic acid duplex, or DNA-RNA hybrid;

wherein the dotted line represents a chemical linkage joining B and A, provided that if B is purine, the linkage is attached to the 9-position of the purine, if B is 7-deazapurine, the linkage is attached to the 7-position of the deazapurine, and if B is pyrimidine, the linkage is attached to the 5-position of the pyrimidine; and

wherein each of x, y and z represents

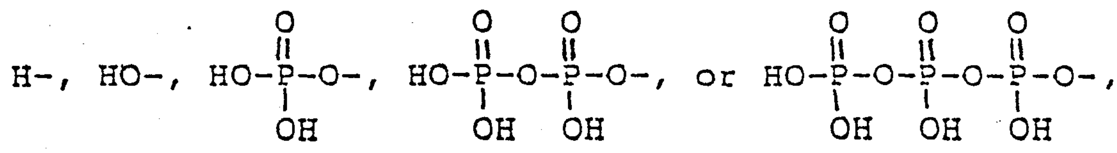

are widely useful as probes in biomedical research and recombinant DNA technology.

Particularly useful are compounds encompassed within this structure which additionally have one or more of the following characteristics: A is non-aromatic; A is at least $C_5$; the chemical linkage joining B and A includes an α-olefinic bond; A is biotin or iminobiotin; and B is a pyrimidine or 7-deazapurine.

EP-A-0 063 879 also discloses compounds having the structure:

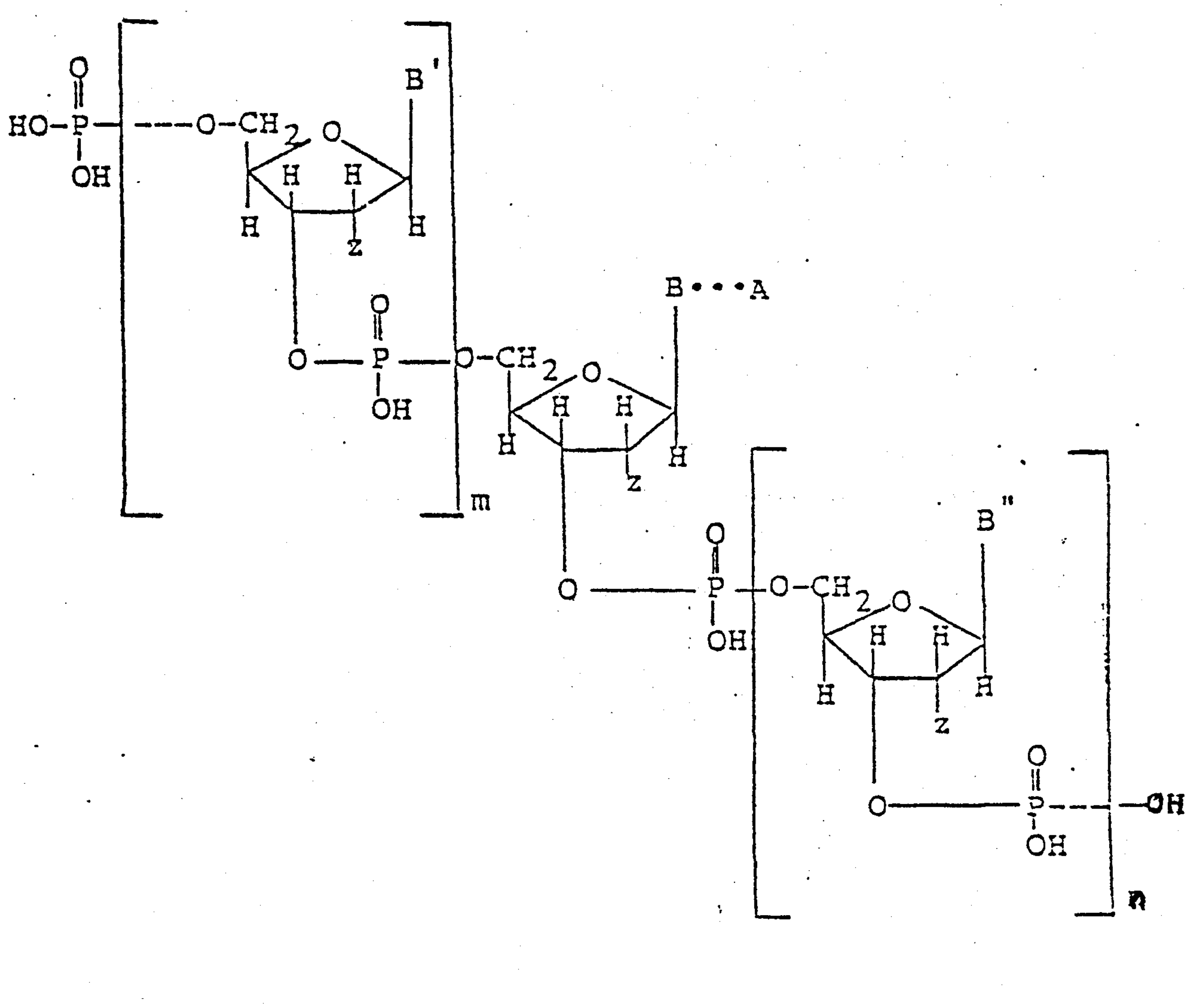

wherein each of B, B', and B" represents a purine, 7-deazapurine, or pyrimidine moiety covalently bonded to the $C^{1'}$-position of the sugar moiety, provided that whenever B, B', or B" is purine or 7-deazapurine, it is attached at the $N^9$-position of the purine or 7-deazapurine, and whenever B, B', or B" is pyrimidine, it is attached at the $N^1$-position;

wherein A represents a moiety consisting of at least three carbon atoms which is capable of forming a detectable complex with a polypeptide when the compound is incorporated into a double-stranded duplex formed with a complementary ribonucleic or deoxyribonucleic acid molecule;

wherein the dotted line represents a chemical linkage joining B and A, provided that if B is purine, the linkage is attached to the 8-position of the purine, if B is 7-deazapurine, the linkage is attached to the 8-position of the deazapurine, and if B is pyrimidine, the linkage is attached to the 5-position of the pyrimidine;

wherein z represents H- or HO-; and

wherein m and n represent integers from 0 up to about 100,000.

These compounds can be prepared by enzymatic polymerization of a mixture of nucleotides which include the modified nucleotides of this invention. Alternatively, nucleotides present in oligo- or polynucleotides may be modified using chemical methods.

The chemically-labeled or modified nucleotides described in the above-referred PNAS article and in EP-A-0 063 879 as indicated hereinabove is the structure:

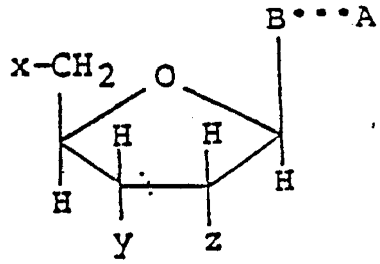

wherein B, A the dotted line and x,y and z are as defined above.

Although according to EP-A-0 063 879 in principal, all compounds encompassed within the above structural formula may be prepared and used, certain of the compounds are more readily prepared or used or both, and therefore are preferred.

Thus, although according to EP-A-0 063 879 purines, pyrimidines and 7-deazapurines are in principal useful, pyrimidines and 7-deazapurines are preferred since purine substitution at the 8-position tends to render the nucleotides ineffective as polymerase substrates. Thus, although modified purines are useful in certain respects, they are not as generally useful as pyrimidines and 7-deazapurines. Moreover, pyrimidines and 7-deazapurines useful in that invention must not be naturally substituted at the 5- or 7- positions, respectively. As a result, certain bases, such as thymine, 5-methylcytosine, and 5-hydroxymethylcytosine, are not useful. Preferred bases are cytosine, uracil and deazaguanine.

A may be any moiety which has at least three carbon atoms and is capable of forming a detectable complex with a polypeptide when the modified nucleotide is incorporated into a double-stranded duplex containing either deoxyribonucleic or ribonucleic acid.

A therefore may be any ligand which possesses these properties, including haptens which are only immunogenic when attached to a suitable carrier, but are capable of interacting with appropriate antibodies to produce complexes. Examples of moieties which are useful according to EP-A-0 063 879 include:

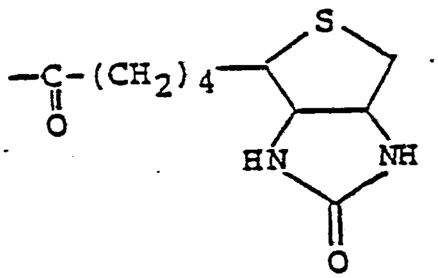

;

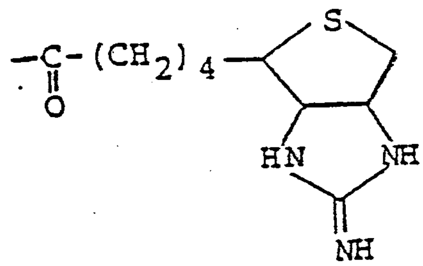

;

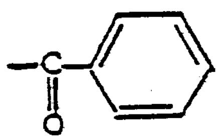

;

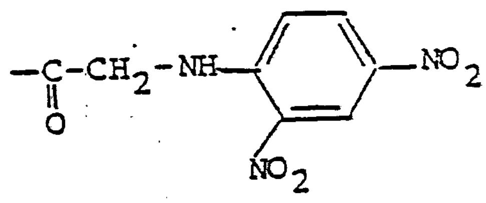

;

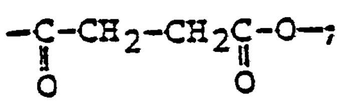

;

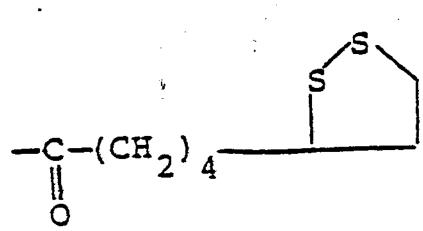

; and

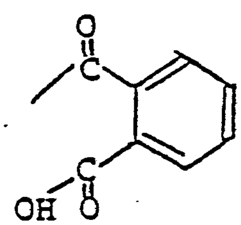

Of these, the preferred A moieties are biotin and iminobiotin.

Moreover, since aromatic moieties tend to intercalate into a base-paired helical structure, it is preferred in EP-A-0 063 879 that the moiety A be nonaromatic. Also, since smaller moieties may not permit sufficient molecular interaction with polypeptides, it is preferred that A be at least $C_5$ so that sufficient interaction can occur to permit formation of stable complexes. Biotin and iminobiotin are said to satisfy both of these criteria.

According to EP-A-0 063 879 the linkage or group joining moiety A to base B may include any of the well known bonds, including carbon-carbon single bonds, carbon-carbon double bonds, carbon-nitrogen single bonds, or carbon-oxygen single bonds. However, it is generally preferred that the chemical linkage include an olefinic bond at the α-position relative to B. The presence of such an α-olefinic bond serves to hold the moiety A away from the base when the base is paired with another in the well known double-helix configuration. This permits interaction with polypeptide to occur more readily, thereby facilitating complex formation. Moreover, single bonds with greater rotational freedom may not always hold the moiety sufficiently apart from the helix to permit recognition by and complex formation with polypeptide.

According to EP-A-0 063 879 it is even more preferred that the chemical linkage group be derived from a primary amine, and have the structure $-CH_2-NH-$, since such linkages are easily formed utilizing any of the well known amine modification reactions. Examples of preferred linkages derived from allylamine and allyl-(3-amino-2-hydroxy-1-propyl) ether groups have the formulae $-CH=CH-CH_2-NH-$ and $-CH=CH-CH_2-O-CH_2-\underset{\displaystyle OH}{\underset{|}{CH}}-CH_2-NH-$,

respectively.

Although these linkages are preferred, others can be used, including particularly olefin linkage arms with other modifiable functionalities such as thiol, carboxylic acid, and epoxide functionalities.

The linkage groups are attached at specific positions, namely, the 5-position of a pyrimidine, the 8-position of a purine, or the 7-position of a deazapurine. Substitution at the 8-position of a purine does not produce a modified nucleotide which is useful in all the methods discussed in EP-A-0 063 879. It may be that the 7-position of a purine, which is occupied by a nitrogen atom, could be the point of linkage attachment. However, the chemical substitution methods employed up to that time were not suitable for this purpose.

The letters x, y, and z represent groups attached to the 5', 3' and 2' positions of the sugar moiety. They may be any of

H-, HO-, 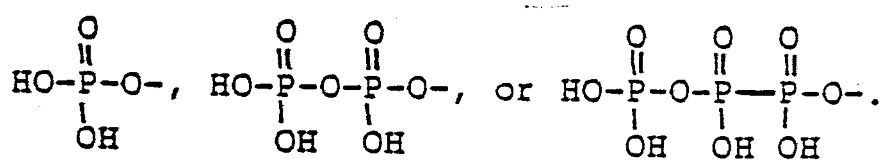

or

Although conceivable, it is unlikely that all of x, y, and z will simultaneously be the same. More likely, at least one of x, y, and z will be a phosphate-containing group, either mono-, di-, or tri-phosphate, and at least one will be HO- or H-. As will be readily appreciated, the most likely identity of z will be HO- or H- indicating ribonucleotide or deoxyribonucleotide, respectively. Examples of such nucleotides include 5'-ribonucleoside monophosphates, 5'-ribonucleoside diphosphates, 5'-deoxyribonucleoside triphosphates, 5'p-ribonucleoside-3'p, and 5'p-deoxyribonucleoside-3'p. More specific examples include modified nucleotides of this type in which A is biotin or iminobiotin, the chemical linkage is

0124124

$-CH=CH-CH_2-NH-$ or 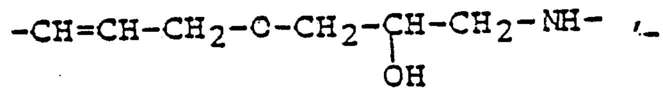

and B is uracil or cytosine.

The general synthetic approach adopted for introducing the linker arm and probe moiety onto the base is discussed thereinabove. (See especially, J.L. Ruth and D.E. Bergstrom, and M.K. Ogawa, J. Amer. Chem. Soc. 100, 8106, 1978; and C.F. Bigge, P. Kalaritis, J.R. Deck and M.P. Mertes, J. Amer. Chem. Soc. 102, 2033, 1980.) However, the olefin substituents employed herein have not been used previously. To facilitate attachment of probe moiety A, it is stated in EP-A-0 063 879 as being particularly desirable to employ olefins with primary amine functional groups, such as allylamine [AA] or allyl-(3-amino-2-hydroxy-1-propyl) ether [NAGE], which permit probe attachment by standard amine modification reactions, such as,

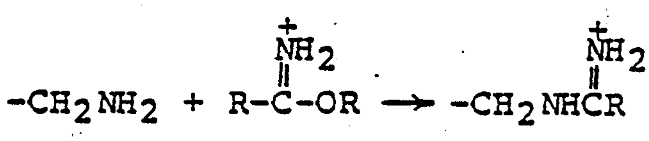

Imidate

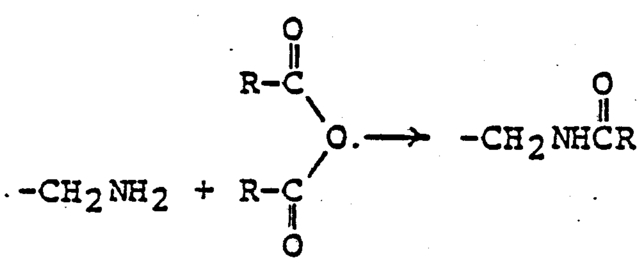

Anhydride

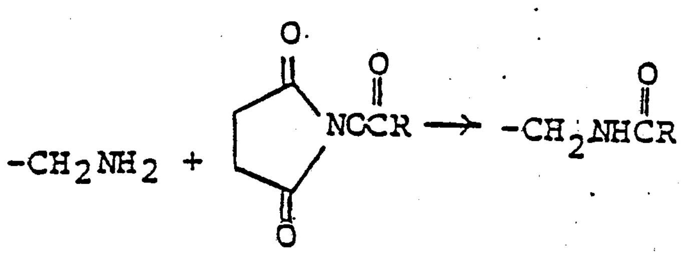

NHS-ester (N-hydroxysuccinimide)

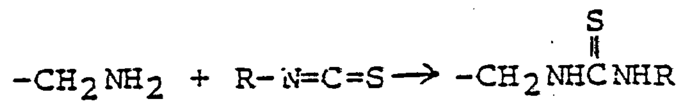

Isothiocyanate

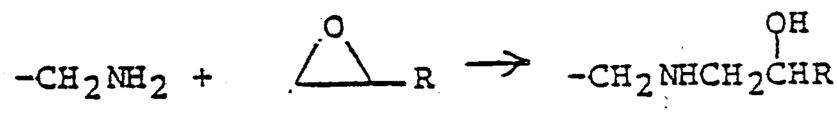

Epoxide

Because of ease of preparation, it is preferable according to EP-A- 0 063 879 to use NHS-esters for probe addition. However, olefin linker arms with other modifiable functional groups, such as thiols, carboxylic acids, epoxides, and the like, can also be employed. Furthermore, both linker arm and probe can be added in a single-step if deemed desirable.

Having the biotin probe directly attached to the nucleotide derivatives that are capable of functioning as enzyme substrates offers considerable versatility, both in the experimental protocols that can be performed and in the detection methods (microscopic and non-microscopic) that can be utilized for analysis. For example, biotin nucleotides can be introduced into polynucleotides which are in the process of being synthesized by cells or crude cell extracts, thus making it possible to detect and/or isolate nascent (growing) polynucleotide chains. Such a procedure is impossible to do by any direct chemical modification method. Furthermore, enzymes can be used as reagents for introducing probes such as biotin into highly selective or site-specific locations in polynucleotides; the chemical synthesis of similar probe-modified products would be extremely difficult to achieve at best.

Further, there are disclosed in EP-A-0 097 373 modified non-radioactive chemically-labeled nucleotides wherein the nucleotides modified such as at the 5-position of pyrimidine or the 7-position of purine, preparatory for the preparation of nucleotide probes therefrom suitable for attachment to or incorporation into DNA or other nucleic acid materials. In the preparation of such modified nucleotides, the nucleotides, i.e. nucleic acids, preferably are modified in a non-disruptive manner such that the resulting modified nucleotides are capable of incorporation into nucleic acids and once incorporated in nucleic acids, the modified nucleotides do not significantly interfere with the formation or stabilization of the double helix formed of the resulting nucleic acids containing the modified nucleotides. The non-disruptive modification of nucleotides and nucleic acids incorporating such modified nucleotides is in contrast with those modifications of nucleotides which are characterized as a disruptive modification in the sense that the resulting disruptively modified nucleotides and nucleic acids containing the same block proper double helix formation. In the practices of EP-A-0 097 373 , the nucleotides are desirably modified at the 5-position of the pyrimidine or the 7-position of the purine. The nucleotides so modified are non-disruptively modified and nucleic acids containing such nucleotides are capable of forming a double helix arrangement.

Broadly, in another aspect of the practices of EP-A-0 097 373 , various methods are useful for the tagging or labeling of DNA in a non-disruptive manner. For example, biotin is added on the end of a DNA or RNA molecule. The addition of biotin is accomplished by addition of a ribonucleotide. The 3',4' vicinal hydroxyl groups are oxidized by periodate oxidation and then reduced by a

borohydride in the presence of biotin hydrazide. Alternatively, carbodiimide can also be used to couple biotin to the aldehyde group.

Another technique for tagging nucleic acid material such as DNA or RNA involves the addition of a large marker to the end of a DNA or RNA molecule. One example of this technique is the addition of a molecule, e.g. lysylglycine, where the amino groups are tagged with biotin. Another example would be to follow the procedure set forth hereinabove but employing carbodiimide as the crosslinking agent. Still another example of this technique would be to produce a biotinylated dA:dU double helical polymer and to ligate this polymer to the probe prepared in accordance with EP-A-0 097 373.

Another technique for tagging DNA in a non-disruptive manner involves the isolation of dPyrTP having a putricine or spermidine on the 5-position from PS16 or phage-infected cells. If desired, dPyrTP is made from phage DNA and phosphorylated to dPyrTP followed by modification of the polyamine side chain by means of standard nucleophilic reagent NHS-biotin.

Another technique for tagging DNA in a non-disruptive manner involves the addition of glucose to 5-hydroxymethylcytosine (5 HMC) in DNA using T4 phage glycosylating enzymes followed by screening by means of a lectin-based assay.

Still another method for tagging DNA in a non-disruptive manner involves 5-HMC-triphosphate made from the hydrolysis of T4-DNA followed by phosphorylation of the 5HMCMP to 5 MMCTP. 5 HMCTP is then incorporated into DNA using polymerase I. Thus, any DNA can be modified to have non-disruptively incorporated therein 5 HMC.

according to EP-A-0097373

A method for tagging DNA in a mildly disruptive manner involves reacting nucleic acids in the double helical form with alkylating reagents as for example benz(o)pyrene diol epoxide or aflatoxin. Under appropriate conditions of the $N^2$ group of guanine, the $N^4$ group of adenosine or the $N^4$ group of cytosine are alkylated. These modified nucleotides can be used as linking arms for the addition of a reporter molecule such as biotin.

These specially modified nucleotides suitable as non-radioactive chemical labels for DNA probes or DNA material as described in EP-A- 0 097 373
can be broadly characterized and described as phosphoric acid P moiety, a sugar or monosaccharide S moiety, a base B moiety, a purine or a pyrimidine and a signalling chemical moiety Sig covalently attached thereto, either to the P, S or B moiety.

Of special interest are those nucleotides having a general formula

$$P - S - B - Sig$$

wherein P is the phosphoric acid moiety including mono-, di-, tri-, or tetraphosphate, S the sugar or monosaccharide moiety, B the base moiety, either a purine or a pyrimidine. The phosphoric acid moiety P is attached at the 3' and/or the 5' position of the S moiety when the nucleotide is a deoxyribonucleotide and at the 2', 3' and/or 5' position when the nucleotide is a ribonucleotide. The base B moiety is attached from the N1 position of the N9 position to the 1' position of the S moiety when the base moiety is a pyrimidine or a purine, respectively. The Sig moiety is covalently attached to the B moiety of the nucleotide and when so attached is capable of signalling itself or makes itself self-detecting or its presence known and desirably or preferably permits the incorporation of the resulting nucleotide P - S - B - Sig into or to form a double-stranded helical DNA or RNA or DNA-RNA hybrid and/or to be detectable thereon.

Another special nucleotide in accordance with EP-A-0 097 373 is characterized by the general formula:

Sig
|
P - S - B

Such nucleotides would be characterized as ribonucleotides. The phosphoric acid moiety is attached at the 2', 3' and/or 5' position of the sugar S moiety and the base B being attached from the N1 position or the N9 position to the 1' position of the sugar S moiety when said base is a pyrimidine or a purine, respectively. The Sig chemical moiety is covalently attached to the sugar S moiety is capable of signalling itself or making itself self-detecting or its presence known and preferably permits the incorporation of the ribonucleotide into its corresponding double-stranded RNA or a DNA-RNA hybrid.

Sig
|
Such nucleotides P - S - B desirably have the Sig chemical moiety attached to the C2' position of the S moiety or the C3' position of the S moiety.

Still further, nucleotides in accordance with the practices of EP-A-0 097 373 include the nucleotides having the formula

Sig
|
P - S - B

wherein P is the phosphoric acid moiety, S the sugar moiety and B the base moiety. In these special nucleotides, the P moiety is attached to the 3' and/or the 5' position of the S moiety when the nucleotide is deoxyribonucleotide and at the 2', 3' and/or 5' position when

the nucleotide is a ribonucleotide. The base B is either a purine or a pyrimidine and the B moiety is attached from the N1 or the N9 position to the 1' position of the sugar moiety when said B moiety is a pyrimidine or a purine, respectively. The Sig chemical moiety is covalently attached to the phosphoric acid P moiety via the chemical linkage

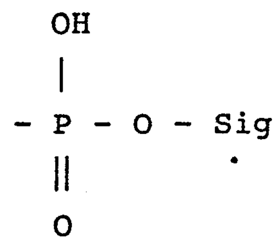

,

said Sig, when attached to said P moiety being capable of signalling itself or making itself self-detecting or its presence known and desirably the nucleotide is capable of being incorporated into a double-stranded polynucleotide, such as DNA, RNA or DNA-RNA hybrid and when so incorporated therein is still self-detecting.

It is pointed out that the special nucleotides in accordance with the practices of EP-A-0 097 373 described or defined hereinabove by the general formula P - S - B - Sig, also include nucleotides wherein the Sig chemical moiety is covalently attached to the B moiety at the $N^6$ or 6-amino group position when the B moiety is adenine or the $N^2$ or 2-amino group position when the B moiety is guanine or the $N^4$ or 4-amino group position when the B moiety is cytosine. The resulting nucleotides containing the Sig moiety attached thereto are capable of signalling themselves or making themselves self-detecting or their presence known and being detectable is a double-stranded or DNA, RNA or DNA-RNA hybrid.

By way of summary, as indicated hereinabove with respect to the make up of the various special nucleotides in accordance with EP-A-0 097 373, the special nucleotides can be described as comprising a phosphoric acid moiety P, a sugar moiety S and a base moiety B, a purine or pyrimidine, which combination of P-S-B is well known with respect to and defines nucleotides, both deoxyribonucleotides and ribonucleotides. The nucleotides are then modified by having covalently attached thereto, to the P moiety and/or the S moiety and/or the B moiety, a chemical moiety Sig. The chemical moiety Sig so attached to the nucleotide P-S-B is capable of rendering or making the resulting nucleotide, now comprising P-S-B with the Sig moiety being attached to one or more of the other moieties, self-detecting or signalling itself or capable of making its presence known per se, when incorporated into a polynucleotide, especially a double-stranded polynucleotide, such as a double-stranded DNA, a double-stranded RNA or a double-stranded DNA-RNA hybrid. The Sig moiety desirably should not interfere with the capability of the nucleotide to form a double-stranded polynucleotide containing the special Sig-containing nucleotide in accordance with this invention and, when so incorporated therein, the Sig-containing nucleotide is capable of detection, localization or observation.

The Sig moiety employed in the make up of the special nucleotides of EP-A-0 097 373 could comprise an enzyme or enzymatic material, such as alkaline phosphatase, glucose oxidase, horseradish peroxidase or ribonuclease. The Sig moiety could also contain a fluorescing component, such as fluorescein or rhodamine or dansyl. If desired, the Sig moiety could include a magnetic component associated or attached thereto, such as a magnetic oxide or magnetic iron oxide, which would

make the nucleotide or polynucleotide containing such a magnetic-containing Sig moiety detectable by magnetic means. The Sig moiety might also include an electron dense component, such as ferritin, so as to be available by observation. The Sig moiety could also include a radioactive isotope component, such as radioactive cobalt, making the resulting nucleotide observable by radiation detecting means. The Sig moiety could also include a hapten component or per se be capable of complexing with an antibody specific thereto. Most usefully, the Sig moiety is a polysaccharide or oligosaccharide or monosaccharide, which is capable of complexing with or being attached to a sugar or polysaccharide binding protein, such as a lectin, e.g. Concanavilin A. The Sig component or moiety of the special nucleotides could also include a chemiluminescent component.

As indicated in accordance with the practices of EP-A-0 097 373, the Sig component could comprise any chemical moiety which is attachable either directly or through a chemical linkage or linker arm to the nucleotide, such as to the base B component therein, or the sugar S component therein, or the phosphoric acid P component thereof.

The Sig component of the nucleotides in accordance with EP-A-0 097 373 and the nucleotides and polynucleotides incorporating the nucleotides containing the Sig component are equivalent to and useful for the same purposes as the nucleotides described in EP-A-0 063 879. More specifically, the chemical moiety A described in EP-A-0 063 879 is functionally the equivalent of the Sig component or chemical moiety of the special

nucleotides of EP-A-0 097 373 . Accordingly, the Sig component or chemical moiety of nucleotides of EP-A-0 097 373 can be directly covalently attached to the P, S or B moieties or attached thereto via a chemical linkage or linkage arm as described in EP-A-0 097 373, as indicated by the dotted line connecting B and A of the nucleotides of EP-A-0 063 879 . The various linker arms or linkages identified in EP-A-0 063 879 applicable to and useful in the preparation of the special nucleotides of EP-A-0 097 373.

A particularly important and useful aspect of the special nucleotides of EP-A-0 097 373 is the use of such nucleotides in the preparation of DNA or RNA probes. Such probes would contain a nucleotide sequence substantially matching the DNA or RNA sequence of genetic material to be located and/or identified. The probe would contain one or more of the special nucleotides of this invention. A probe having a desired nucleotide sequence, such as a single-stranded polynucleotide, either DNA or RNA probe, would then be brought into contact with DNA or RNA genetic material to be identified. Upon the localization of the probe and the formation of a double-stranded polynucleotide containing the probe and the matching DNA or RNA material to be identified, the resulting formed double-stranded DNA or RNA-containing material would then be observable and identified. A probe in accordance with EP-A-0 097 373 may contain substantially any number of nucleotide units, from about 5 nucleotides up to about 500 or more, as may be required. It would appear that 12 matching, preferably consecutive, nucleotide units would be sufficient to effect an identification of most of the DNA or RNA material to be investigated or identified, if the 12 nucleotide sequence of the probe matches a corresponding cooperative sequence in the DNA

or RNA material being investigated or to be identified. As indicated, such probes may contain one or more of the special Sig-containing nucleotides, preferably at least about one special nucleotide per 5-10 of the nucleotides in the probe.

In the practices of this invention it is preferred to employ biotinylated probes as described hereinabove or glycosylated probes. Other chemically-labeled probes are, however, usefully employed in the practices of this

invention. For example, the chemical label can be an antigen which can then be located with an appropriate antibody labeled with biotin. The chemically-labeled probes as indicated hereinabove, must be capable of hybridizing or forming a double-stranded duplex with its complementary or matching DNA or RNA genetic material to be identified and/or located. Desirably, the chemical label moiety, when incorporated in a nucleotide and the nucleotide then employed for nick translation to prepare a clone or a complementary copy of the probe desired, must not interfere with the hybridization of the thus-chemically-labeled probe with the genetic material being investigated. Also, the chemical label moiety of the probe may, if desired, be terminally attached to an unlabeled probe in which instance the chemical label moiety or the chemically-labeled nucleotide must be capable of being used as a substrate for the terminal labeling of the probe, such as a substrate for terminal transferase for attachment to a polynucleotide or DNA probe. As another alternative, the chemical label moiety may be attached to an unlabeled probe via a universal detection system as described in co-pending, co-assigned Patent Application Serial No. 491,929, filed May 5, 1983 entitled "Assay Method Utilizing Polynucleotide Sequences". This patent application discloses a method of indirectly chemically labeling a

probe. Such method comprises providing a polynucleotide sequence attached to the probe. The chemical label moiety is attached to the probe by incorporating a chemical label moiety into a polynucleotide sequence that is capable of hybridizing with the polynucleotide sequence which is attached to the probe.

Alternatively, polynucleotide probes in accordance with this invention can be prepared by chemically modifying or adding to the terminal portion of the probe, such as by fixing thereto a segment of homogeneous nucleotides, such as a length of Poly A or a length of amino acids, such as polylysine or, indeed, any terminal label which may be usefully employed as a marker, such as an antigenic marker, for locating the resulting hybridized probe.

After hybridization of the target genetic material or DNA or RNA fragment with the chemically-labeled probe the hybrid is located by means of suitable reagent material which attaches itself or fixes itself to the chemical label moiety of the chemically-labeled probe. Additionally, the reagent so employed in the practices of this invention for attachment to or fixing to the chemical label moiety of the chemically-labeled probe must also be effective upon contact with another reagent, such as a chromogen, to produce a visual or visible mark or indication of the chemically-labeled nucleotide. In the practices of this invention it is preferred to employ an enzyme complex for attachment to the chemically-labeled probe. For example, when the chemically-labeled probe is biotinylated polynucleotide or contains biotin as the chemical label moiety, an enzyme complex comprising streptavidin-biotinylated enzyme is effective, since the streptavidin-biotinylated enzyme complex would readily attach itself to the biotin

moiety of the biotinylated probe via attachment of the streptavidin to the biotin moiety of the chemical label of the chemically-labeled probe. Also useful would be a lectin-enzyme complex for use in connection with a glycosylated labeled probe, such as a DNA probe having glycosylated groups attached thereto or glycosylated nucleotides making up the probe. Enzymes useful for the detection of the chemically-labeled probe in accordance with the practices of this invention include such enzymes as acid phosphatase, alkaline phosphatase, horseradish peroxidase, glucose oxidase and beta-galactosidase. These enzymes in assocation with a suitable chromogen, after attachment of the enzyme to the chemical label moiety of the hybridized probe, are capable of producing a visible or colored insoluble product or precipitate which would readily signal the presence of the formed hybrid containing the chemical label probe and the genetic material of interest. Also, the Gomori procedure, as described in "Enzyme Histochemistry" by M.S. Burnstone, Academic Press, 1962, which is herein incorporated by reference, can be utilized to produce a visible or colored insoluble product or precipitate. The Gomori procedure utilizes alkaline phosphatase and its chromogen to generate free phosphate which is precipitated as a white compound with calcium. This difficult to visualize precipitate is rendered more visible by contacting such precipitate with cobolt chloride. This results in a purple precipitate. This precipitate can be rendered even more visible by contacting such precipitate with ammonia sulfide which results in a black precipitate. Listed in Table I herein are those enzymes and associated chromogens which are useful in the practices of this invention.

TABLE I

CHROMOGEN (insoluble product)

| ENZYME | |
|---|---|
| 1. Alkaline phosphatase | 6-bromo-3-hydroxy-2-naphthyl-0 anisidine phosphate + Fast Violet B salt. |
| | 2,2'-dimethyl-3-hydroxy-2-naph-thanilide phosphate + Fast Red salt. |
| | flavone-3-disphosphate ammonium salt + $AlCl_3$(F) |
| | 5-bromo-4-chloro-3-indolylphosphate + tetrazolium, nitro BT. |
| | glycerol phosphate (for use in Gomori procedure) |
| 2. Horseradish peroxidase | $H_2O_2$ + diaminobenzidine<br>$H_2O_2$ tetramethylbenzidine |
| 3. Glucose oxidase | glucose + thiazolyl blue |
| 4. B-galactosidase naphthol | 2-(Beta-D-galactosidoxy) AS-LC + Fast Violet B salt. |
| | Naphthol-AS-BL-B-O-galactopyranoside + Fast Blue BBN salt. |
| 5. Acid phosphatase | Naphthol-AS-MX phosphate + Fast violet B salt. |

The preparation of chemical reagents or enzyme complexes useful in accordance with the practices of this invention to signal the presence of the hybridized chemically-labeled probes in described in co-pending, cò-assigned patent application Serial No. 486,924, filed April 20, 1983 entitled "Complexing of Biologically Active or Functional Compounds and Methods of preparaing and Utilizing Same".

The visual or visible mark or indication of the chemically labeled probe can also be produced by utilizing the reaction of starch and iodine, which results in the formation of a strong blue/black color. The starch/iodine system is very safe and economical. This system can be utilized by employing a chemical component which attaches itself or fixes itself to the chemical label moiety of the chemically labeled probe. Such chemical component must also be effective upon contact with a suitable reagent to produce a reaction product that is capable of oxidizing iodide to iodine. Therefore, when a soluble iodide, such as potassium iodide or sodium iodide, and starch is added to such system, the reaction product will oxidize the iodide to iodine which will then react with the starch to generate a strong blue/black color. An example of a chemical component that can produce such reaction product is a chemical component that contains any of the oxidase enzymes, such as glucose oxidase and lipoxidase. Such enzyme can be attached to the chemically labeled probe via an enzyme complex, as described hereinabove. When such oxidase enzyme is treated with a suitable reagent, such as beta D-glucose for glucose oxidase or linoleic acid for lipoxidase, a reaction product is hydrogen peroxide, which is capable of oxidizing iodide to iodine. It is believed that this indirect detection

system can be utilized for generating a reaction product ($H_2O_2$) that is capable of interacting with a second chemical component (iodide) to convert the second reaction product to a form that is capable of interacting with a third chemical component (starch) to produce a colored or visible product.

The starch/iodine system can be utilized in either an insoluble assay system, for example, to produce an insoluble color precipitate or in a soluble assay system, for example, an ELISA assay. An insoluble color precipitate can be produced by utilizing insoluble starch or by fixing soluble starch to the same substrate that the chemically labeled probe is fixed to or by utilizing a second substrate which has the soluble starch fixed thereto; the second substrate is then placed in intimate contact with the substrate to which the chemically labeled probe is fixed. It should be noted that the fixing of the soluble to starch to a second substrate; i.e., a substrate other than that to which the chemically labeled probe is fixed, is a way to convert a soluble assay system which can form a soluble or gaseous reaction product which can further react with another chemical component that is fixed to a second substrate to an insoluble assay system. Furthermore, the fixing of a chemical component to a second substrate results in the precipitate being produced on such substrate rather than the substrate to which the chemically labeled probe is fixed. Thus, the precipitate is formed away from the enzyme and therefore will eliminate the potential for the precipitate to block and thereby, hinder the activity of the enzyme which is fixed to the chemically labeled probe. This results in a more intense color than when the insoluble color precipitate is formed on the substrate to which the enzyme is fixed.

It should be noted that a single sample of genetic material to be invesigated can be analyzed for more than one polynucleotide sequence by utilizing more than one probe. However, when this multiple analysis is carried out, it is essential to be able to distinguish the insoluble color precipitates that are formed from each probe. This can be carried out by employing either probes with different chemical labels and/or probes that employ the same chemical label, but one probe is labeled directly, e.g., biotinylated or glycosylated, and the other is labeled indirectly. The two or more probes can be utilized simultaneously to hybridize with their corresponding polynucleotide sequence. The hybridized probes can be located simultaneously if chromogens are utilized that generate different colors or sequentially if chromogens that generate the same color are utilized, providing that a record of the location of the first probe is recorded. For example, a biotinylated probe and a glycosylated probe can be utilized simultaneously to detect two different polynucleotide sequences. After detection, the probe is contacted with the appropriate enzyme complex which is then followed by the contacting such complex with an appropriate chromogen sequentially or simultaneously so as to generate the same visual or visible mark or a different visual or visible mark respectively. Alternatively, a biotinylated probe and a probe having a polynucleotide sequence attached thereto can be utilized simultaneously to detect their complementary polynucleotide sequence. The biotinylated probe can be located with the appropriate enzyme complex. The probe having the polynucleotide sequence attached thereto can then be located with a polynucleotide sequence labeled with biotin and complementary to the polynucleotide sequence attached to the probe. Such biotin chemical label can then be located by the same manner as the biotinylated probe.

Indicative of the efficacy of a chemically-labeled probe in accordance with the practices of this invention in the above-indicated molecular biology techniques, dot blots, Southern and colony hybridization and the like, chemically-labeled probes in accordance with this invention have been employed for the detection of single genes in Southern tranfer of genomic E. Coli DNA. In this work BAM H-1 E. Coli genomic DNA preparation from a $lac^+$ strain (BL 15) and from a strain deleted for lac (2089) were separated on agarose gels and transferred to nitrocellulose membranes. The nitrocellulose-bound DNA was hybridized to biotinylated plasmid probe containing 200 base pairs of the lac separator-promoter region. Detection was carried out employing a streptavidin-biotinylated horseradish peroxidase complex. The lac DNA bands appeared in all lanes to which as little as 1.0 microgram of DNA had been run. These techniques employing the chemically-labeled DNA probes in accordances with this invention have been extended to the detection of single gene copies of genomic blots of human DNA. In this work a biotinylated plasmid probe carrying a 4.4 kilobase betaglobin gene insert was hybridized to blots of BAM H-1 restricted HeLa cell DNA. Detection was also carried out employing streptavidin-biotinylated horseradish peroxidase in lanes in which as little as 1.0 microgram of restricted genomic DNA had been run.

As mentioned hereinabove, chemically labeled probes in accordance with this invention had been employed in colony hybridization procedures based on the Grunstein-Hogness procedure, see also the article entitled "Colony Hybridization" mentioned herein. Detection was accomplished by employing

0124124

streptavidin-biotinylated horseradish peroxidase. The background noise was very low, thereby providing easy identification of "positive colonies". The techniques of genetic material analysis employing the chemically-labeled probes in accordance with this invention provide for better resolution than is attainable with radioactive phosphorous-labeled probes so that positive colonies can be easily identified even on crowded plates.

An an option in the practices of this invention, if no hybridized genetic material is located by means of a suitable reagent, then the negative result can be confirmed. If the techniques for analysis of genetic material, e.g. colony hybridization, southern, northern and western blots, dot blots, plaque lifts, employing the chemically labeled probe does not produce a visual or visible mark or indication of the chemically labeled genetic material, then a due to the fact that no genetic material was fixed to the substrate rather than the absence of the target genetic material. This can be verified, i.e., confirmation of the negative result, by detecting to verify the presence of the genetic material.

Detecting to verify the presence of the genetic material can be accomplished by any means utilized in the art for such purpose. Nonlimiting examples include chemical counterstaining, using antibodies against the genetic material and chemical reactions that detect genetic material. Suitable chemical stain for counterstaining the genetic material include ethidium bromide, acridine orange, the Feulgen nuclear reaction for DNA as described in Feulgen, R, and Rossenbeck, H., Zeitschrift Physiology Chemistry, 135, 203 and the naphthoic acid hydrazide Feulgen method for DNA as described in Pearse,

A.G.E., Quarterly Journal of Microscopical Science, 92, 202 (1951), with ethidium bromide being preferred. The procedure for the use of antibodies against genetic material is described Andrezeiewski, C. et.al., J. Immunol.126, 226-231 (1981) and Lipps, H.J., Cell, 32, 435-441. Suitable chemical reactions include the Burton method as described in Burton, Biochem, J., 62: 315-325 (1957) and Giles, K.W., and A. Meyers, Nature (London) 206, 93.

It should be noted that the detection to verify the presence of the genetic material can be carried out at any step, or at even more than one step, in the techniques in the practices of this invention. However, it is preferred to detect to verify the presence of genetic material after contact with the chemically labeled probe, so as to prevent the possibility of interference with hybridization. It is most preferred to carry out such detection after attempting to locate the hybridized genetic material by means of a suitable reagent. This is due to the fact that there is no reason to carry out such detection when there is a positive result, i.e. the chemically labeled probe does produce a visual or visible mark or indication of the chemically labeled genetic material and to prevent the possibility of interference with locating the hybridized genetic material. Also, it should be noted that such detection is applicable to protein and peptide analysis. This is accomplished by any method utilized in the art to detect such material.

Although emphasis with respect to the practices of this invention has been placed upon the analysis of genetic material, such as DNA or RNA, the practices of this invention as already indicated are also applicable to protein or peptide analysis, such as by the Western blot

techniques already mentioned. In the practices for this invention employing the Western blot technique for protein or peptide analysis, the material to be analyzed, such as protein, e.g. tissue protein or cellular protein, is solubilized by standard methods, then transferred to a gel and fractionated, such as by gel electrophoresis. The resulting fractionated protein material or peptide material is then transferred to a suitable substrate, e.g. nitrocellulose paper or Pall membrane, and fixed thereto. The fixed fractionated protein material is then treated in accordance with the practices of this invention for detection of the protein or peptide of interest. For example, a biotinylated antibody to the protein or peptide of interest is prepared and applied to the fixed fractionated protein or peptide material on the substrate. Thereupon, an enzyme complex made up of streptavidin-biotinylated enzyme, such as a complex comprising streptavidin-biotinylated alkaline phosphatase, is applied to the biotinylated antibody now fixed or attached to the protein or peptide of interest with resultant fixing of the enzyme complex to the biotinylated antibody. The protein of interest would then be located or defined by application of a chromogen in accordance with the practices of this invention, all as described hereinabove.

Although the binding between biotin and avidin is strong, it is preferred to employ streptavidin in the make-up of the enzyme complex in the practices of this invention. Streptavidin, a biotin binding protein, is produced by Streptomyces avidinii and is similar to egg white avidin. However, streptavidin does not bind to DNA non-specifically. In the practices of this invention as indicated hereinabove, the enzyme complex can be or the streptavidin-biotinylated enzyme complex

0124124

can be used for either in situ detection or visualization on a suitable substrate, such as nitrocellulose paper or a nylon membrane, preferably a nylon membrane that is chemically derivatized such as Pall membrane.

The present invention lends itself readily to preparation of kits comprising one or more of the elements necessary to perform the methods of the present invention. Thus, a kit may comprise a carrier being compartmentalized to receive in close confinement therein one or more container means or series of container means such as test tubes, vials, glass, bottles, syringes, or the like. A first of such container means comprises a chemically labeled nucleotide. The second container means can contain an enzyme complex comprising an enzyme effective upon contact with a chromogen to produce an insoluble color precipitate. A third container means can contain a chromogen effective on contact with said enzyme complex to produce an insoluble color precipitate. The fourth container means can contain enzyme means for terminally labeling a single stranded polynucleotide probe with the chemically labeled nucleotide or for the incorporation of the chemically label nucleotide, such as by nick translation, to produce the chemically labeled polynucleotide probe. Such kit can be utilized to chemically label a polynucleotide probe and then to detect such probe. Also, a kit can be prepared which contains the chemically labeled probe itself, for example, a chemically labeled polynucleotide probe or a chemically labeled protein or polypeptide probe. This kit can contain as the first container means a chemically labeled probe, the second container means can contain an enzyme complex comprising an enzyme effective upon contact with a chromogen to produce an insoluble color precipitate and a third container means which contains a chromogen effective on contact with said enzyme of said enzyme complex to produce an insoluble color precipitate. Each of the above kits can further contain a substrate or matrix which is utilized to fix the probe thereto. For example, the substrate

can be nitrocellulose paper or nylon membrane or a glass slide. Also, each of the above kits can further comprise a container means containing a hybridization solution for the labeled probe, a container means containing a buffer solution for the enzyme of the enzyme complex and a container means containing a buffer solution for the chromogen. It should be noted that depending on the enzyme and chromogen utilized the same buffer solution may be able to be utilized for the enzyme and chromogen.

Preferred embodiments of the invention are explained in detail in the following enumeration:

1. In a method for the analysis of genetic material, such as DNA and/or RNA, wherein the genetic material is denatured, fixed to a substrate and hybridized with a probe, the improvement which comprises employing as said probe a chemically labeled probe, said chemically labeled probe having attached thereto a chemical component effective upon contact with a chromogen to produce an insoluble color precipitate.

2. A method in accordance with item 1 wherein said component is attached to said chemical label after said probe has been hybridized with said genetic material fixed to said substrate.

3. A method in accordance with item 1 wherein said chemical component comprises an enyzme.

4. A method in accordance with item 1 wherein said substrate is selected from the group consisting of glass, glass coated material, plastic, plastic coated material, siliceous material, cellulose paper, denatured cellulose paper, nitrocellulose paper dextran.

5. A method in accordance with item 1 wherein said substrate is or comprises nitrocellulose paper.

6. A method in accordance with item 1 wherein said substrate is or comprises aminobenzyloxymethyl (ABM) paper.

7. A method in accordance with item 1 wherein said substrated is or comprises a nylon membrane.

8. A method in accordance with item 7 wherein said membrane is chemically derivatized.

9. A method in accordance with item 1 wherein said genetic material is or comprises human genetic material.

10. A method in accordance with item 1 wherein said genetic material is or comprises bacterial genetic material.

11. A method in accordance with item 1 wherein said genetic material is or comprises viral genetic material.

12. A method in accordance with item 1 wherein said genetic material is or comprises DNA.

13. A method in accordance with item 1 wherein said genetic material is or comprises RNA.

14. A method in accordance with item 1 wherein said chemically labeled probe is a probe which has said chemical label terminally attached to said probe.

15. A method in accordance with item 1 wherein said chemically labeled probe comprises one or more biotinylated nucleotides.

16. A method in accordance with item 1 wherein said chemically labeled probe comprises one or more glycosylated nucleotides.

17. A method in accordance with item 16 wherein said glycosylated nucleotides are derived from phage T4.

18. A method in accordance with item 1 wherein said chemical component comprises an enyzme selected from the group consisting of acid phosphatase, alkaline phosphatase, horseradish peroxidase, glucose oxidase and beta-galactosidase.

19. A method in accordance with item 1 wherein said chromogen is 6-bromo-3-hydroxy-2-naphthyl-O-anisidine phosphate plus Fast violet B salt; 2,2' -dimethyl-3-hydroxy-2-naphthanilide phosphate plus Fast Red salt; flavone-3-diphosphate ammonium salt plus $AlCl_3$; 5-bromo-4-chloro-3-indolyphosphate plus nitro BT; $H_2O_2$ plus diamino-benzidine; $H_2O_2$ plus tetramethyl benzidine; glucose plus thiazolyl blue; 2-(beta-D-galactosidoxy) naphthol AS-LC plus Fast Violet B salt naphthol -AS-B1-B-O-galactopyranoside plus Fast Blue BBN salt and naphthol AS-MX phosphate + Fast Violet B salt.

20. A method in accordance with item 1 wherein said chemically labeled probe comprises biotinylated DUTP nucleotide component thereof.

21. A method in accordance with item 1 wherein said chemically labeled probe has attached thereto as said chemical component an enzyme complex comprising as a portion thereof a moiety which attaches itself to said chemically labeled probe.

22. A method in accordance with item 21 wherein said chemically labeled probe comprises one or more biotinylated nucleotides and wherein said enyzme complex comprises a streptavidin-biotinylated enzyme, the streptavidin portion of said enzyme complex attaching or fixing itself to the biotin moiety of said biotinylated nucleotides.

23. A method in accordance with item 22 wherein said enzyme of said streptavidin-biotinylated enzyme is horseradish peroxidase.

24. A method in accordance with item 1 which further comprises verifying the presence of undetected genetic material after said chemically labeled probe has been employed to detect said genetic material.

25. A method in accordance with item 24 wherein verification of the presence of undetected genetic material is carried out after said genetic material has been contacted with said probe.

26. A method in accordance with item 1 which further comprises contacting said chemical component with said chromogen to produce an insoluble color precipitate.

27. A method in accordance with item 26 which further comprises verifying the presence of undetected genetic material when no insoluble color precipitate is produced.

28. A method in accordance with item 27 wherein verification of the presence of undetected genetic material is carried out by chemical counterstaining, using antibodies against said genetic material or chemical reactions that detect said genetic material.

29. A method in accordance with item 28 wherein verification of the presence of said genetic material is carried out with ethidium bromide.

30. A kit useful for the analysis of genetic material comprising a chemically labeled nucleotide, an enzyme complex comprising an enzyme effective upon contact with a chromogen to produce an insoluble color precipitate, a chromogen effective contact with said enzyme of said enzyme complex to produce an insoluble color precipitate, and enzyme means for terminally labeling a single stranded polynucleotide probe with said chemically labeled nucleotide or for the incorporation of said chemically labeled nucleotide, such as by nick translation, to produce said probe.

31. A kit in accordance with item 30 wherein said chemically labeled nucleotide is biotinylated nucleotide, wherein enzyme complex comprises streptavidin-biotinylated glucose oxidase and wherein said enzyme means is terminal transferase.

32. A kit in accordance with item 30 wherein said chemically labeled nucleotide is a glycosylated nucleotide, wherein said enzyme complex comprises lectin and said enzyme, the lectin moiety of said enzyme complex being capable of attachment to the glycosyl moiety of said glycosylated nucleotide.

33. A kit in accordance with item 30 comprising additionally paper suitable for the fixing of denatured genetic material thereto.

34. A kit in accordance with item 30 further comprising means for verifying the presence of undetected genetic material.

35. A kit in accordance with item 34 wherein ethidium bromide is employed for verifying the presence of undetected genetic material is ethidium bromide.

36. In a method for the analysis of genetic material, such as DNA or RNA, wherein genetic material is denatured, fixed <u>in situ</u> or to a substrate and hybridized with a probe the improvement which comprises employing as said probe a chemically labeled probe, said chemically labeled probe having attached thereto a chemical component effective upon contact or reaction with a chromogen to produce a colored or visible product or an insoluble color precipitate.

37. A method in accordance with item 36 which further comprises verifying the presence of undetected genetic material after said chemically labeled probe has been employed to detect said genetic material.

38. A method in accordance with item 37 wherein said verification of the presence of said genetic material is carried out after said genetic material has been contacted with said probe.

39. A method in accordance with item 39 which comprises contacting said chemical component with said chromogen to produce a colored visible product or an insoluble color precipitate.

40. A method in accordance with item 39 which comprises verifying the presence of undetected genetic material when no colored or visible product or insoluble color precipitate is produced.

41. A method in accordance with item 40 wherein said verification of the presence of undetected material is carried out by chemical counterstaining, using antibodies against said genetic material or chemical reactions that detect said genetic material.

42. A method in accordance with item 41 wherein verification of the presence of said genetic material is carried out with ethidium bromide.

43. A method for the analysis of protein, protein-like or peptide material which comprises solubilizing said material, subjecting the resulting solubilizing material to fractionation, contacting the resulting fractionerial material with a complex, said complex comprising anted material with a complex said complex comprising an antibody for a selected component of said fractionated material, said complex having attached to the antibody thereof a first reactant material and fixing of said complex via said antibody to said select component, thereupon contacting the resulting complexed fractionated material with a chemical reagent comprising a second reactant

material, said second reactant material being capable of complexing with or attaching to said first reactant material, said chemical reagent also comprising a third reactant material being effective upon contact or reaction with a chromogen to produce a colored or visible product or bringing said chromogen into contact with said chemical reagent to produce said colored or visible product or insoluble color precipitate, thereby eliciting or identifying the presence of said selected component of said fractionated material.

44. A method in accordance with item 43 wherein said solubilized material is fractionated by gel electrophoresis.

45. A method in accordance with item 43 wherein said solubilized material is fractionated by gel electrophoresis and the resulting fractionated material separated and fixed to a substrate.

46. A method in accordance with item 45 wherein said substrate is nitrocellulose paper.

47. A method in accordance with item 45 wherein said substrate is a nylon membrane.

48. A method in accordance with item 45 wherein said complex comprises a biotinylated antibody and wherein said chemical reagent comprises a streptavidin-biotinylated enzyme.

49. A method in accordance with item 48 wherein said enzyme is horseradish peroxidase.

50. In a method for the analysis or detection of a selected biological material, such as DNA, protein or a polypeptide, wherein the biological material is fractionated, fixed to a substrate and detected by a probe which attaches itself to the selected biological material to be determined or improvement which comprises employing as said probe a probe which attaches itself to said selected biological material, said probe being provided with a chemical label moiety which is detectable by contact with a chemical component which attaches itself to said chemical label moiety of said probe and which is effective upon contact with a chromogen to produce a colored or visible product or insoluble color precipitate.

51. A method in accordance with item 50 wherein said biological material comprises DNA or RNA genetic material and wherein said probe comprises a biotinylated or glycosylated nucleotide.

52. A method in accordance with item 50 wherein said biological material is a protein or polypeptide and wherein said probe comprises an antibody to said protein or polypeptide.

53. A method in accordance with item 50 wherein said selected biological material is DNA or RNA genetic material, wherein said probe comprises a biotinylated or glycosylated nucleotide and wherein said chemical component comprises an enzyme complex attachable to the chemical label moiety of said probe and effective upon contact with a chromogen to produce a colored or visible product or insoluble color precipitate.

54. A kit for the analysis of genetic material comprising terminal transferase, a biotinylated or glycosylated nucleotide as a substrate for said terminal transferase an enzyme complex for attachment to said biotinylated of glycosylated nucleotide and a membrane or filter substrate material selected from nitrocellulose paper or nylon membrane.

55. A kit in accordance with item 54 wherein said kit also comprises a chromogen effective upon contact with or in association with said enzyme complex to produce a colored or visible product or insoluble color precipitate.

56. A kit in accordance with item 55 wherein said substrate for terminal transferase is a biotinylated nucleotide said enzyme complex comprises a streptavidin-biotinylated enzyme complex.

57. A kit in accordance with item 56 wherein said biotinylated enzyme is biotinylated horseradish peroxidase.

58. A kit in accordance with item 56 wherein said biotinylated enzyme is biotinylated alkaline phosphatase.

59. A kit for the analysis of genetic material comprising:

A) a chemically labeled probe capable of hybridizing with at least a portion of said genetic material.

B) an enzyme complex attachable to said chemical label, said enzyme complex comprising an enzyme effective on contact with a chromogen to produce an insoluble color precipitate;

C) a chromogen effective on contact with said enzyme of said enzyme complex to produce an insoluble color precipitate.

60. A kit in accordance with item 59 wherein said chemical label is biotin and said enzyme complex comprises a streptavidin-biotinylated enzyme complex.

61. A kit in accordance with item 59 wherein said chemical label is a glycosyl group and said enzyme complex comprises a lectin and said enzyme, wherein said lectin is capable of forming a complex with said glycosyl group.

62. A kit in accordance with item 59 wherein said enzyme is selected from the group consisting of alkaline phosphatase, acid phosphatase, horseradish peroxidase, glucose oxidase and beta galactosidase.

63. A kit in accordance with item 59 further comprising a substrate for fixing or attaching said genetic material thereto.

0124124

64. A kit in accordance with item 59 wherein said genetic material is cytomegalovirus and said chemically labeled probe is a chemically labeled cytomegalovirus probe.

65. A kit in accordance with item 59 wherein said genetic material is Herpes Simplex Virus I and/or II and wherein said chemically labeled probe is a chemically labeled Herpes Simplex Virus I and/or II probe.

66. A kit in accordance with item 59 wherein said genetic material is Hepatitis B and said chemically labeled probe is a chemically labeled Hepatitis B probe.

67. A kit in accordance with item 59 wherein said genetic material is Chlamydia and said chemically labeled probe is a chemically labeled Chlamydia probe.

68. A kit in accordance with item 59 wherein said genetic material is adenovirus and said chemically labeled probe is a chemically labeled adenovirus probe.

69. A kit in accordance with item 59 wherein said genetic material is Hepatitis A and said chemically labeled probe is chemically labeled Hepatitis A probe.

70. A kit in accordance with item 59 wherein said genetic material is Epstein Barr Virus and said chemically labeled probe is a chemically labeled Epstein Barr Virus probe.

71. A kit in accordance with item 59 wherein said genetic material is Neisseria gonorrhoeae and said chemically labeled probe is a chemically labeled Neisseria gonorrhoeae probe.

72. A kit in accordance with item 59 wherein said genetic material is Mycoplasma and said chemically labeled probe is a chemically labeled Mycoplasma probe.

73. A kit in accordance with item 59 wherein said genetic material is Streptococcus A and said chemically labeled probe is a chemically labeled Streptococcus A probe.

74. A kit in accordance with item 59 further comprising a hybridization solution for said labeled probe, a buffer solution for said enzyme and a buffer solution for said chromogen.

75. A kit for the identification of proteins or peptides comprising:

A) a chemically labeled protein or peptide probe;

B) an enzyme complex attachable to said chemical label, said enzyme complex comprising an enzyme effective upon contact with a chromogen to produce an insoluble color precipitate;

C) a chromogen effective on contact with said enzyme of said enzyme complex to produce an insoluble color precipitate.

0124124

76. A kit in accordance with item 75 wherein said chemical label is biotin and said enzyme complex comprises a streptavidin-biotinylated enzyme complex.

77. A kit in accordance with item 75 wherein said chemical label is a glycosyl group and said enzyme complex comprises a lectin and said enzyme, wherein said lectin is capable of forming a complex with said glycosyl group.

78. A kit in accordance with item 75 wherein said enzyme is selected from the group consisting of alkaline phosphatase, acid phosphatase, horseradish peroxidase, glucose oxidase, and beta galactosidase.

79. A kit in accordance with item 75 further comprising a substrate for fixing or attaching said proteins or peptides thereto.

80. A kit in accordance with item 79 wherein said substrate is selected from nitrocellulose paper or nylon membrane.

81. A method for the analysis or detection of a selected biological material such as DNA, protein or a polypeptide, which comprises:

a) fixing said biological material to a substrate;

b) detecting said biological material with a chemically labeled probe;

c) contacting said chemically labeled probe with a chemical component which attaches itself to said chemical label moiety of said probe and is effective on contact with a reagent to produce a reaction product that is capable of oxidizing soluble iodide to iodine;

d) contacting said chemical component with said reagent product that is capable of oxidizing iodide to iodine; and

e) contacting said reaction product with a soluble iodide and starch so as to produce a colored or visible product.

82. A method in accordance with item 81 wherein said chemical component comprises an oxidase enzyme.

83. A method in accordance with item 82 wherein said oxidase enzyme is selected from the group consisting of glucose oxidase and lipoxidase.

84. A method in accordance with item 81 wherein said reagent is selected from the group consisting of glucose and linoleic acid.

85. A method in accordance with item 81 wherein said soluble iodide is selected from the group consisting of potassium iodide and sodium iodide.

86. A method in accordance with item 81 wherein said colored or visible product is an insoluble color precipitate.

87. A method for the detection of a chemically labeled probe which comprises:

(a) contacting said chemically labeled probe with a chemical component which attaches itself to said probe and is effective on contact with a reagent to produce a reaction product that is capable of oxidizing soluble iodide to iodine; and;

(b) contacting said chemical component with said reagaent to produce said reaction product.

(c) contacting said reaction product with a soluble iodide and starch so as to produce a colored or product.

88. A method for the conversion of a soluble assay system for the analysis or detection of a biological material with a chemically labeled probe to an insoluble assay system which comprises:

a) providing a chemically reactive substance fixed to a substrate;

b) contacting said chemically labeled probe with a chemical component which attaches itself to said chemical label moiety of said probe and is effective on contact with a reagent to produce a gaseous reaction product that is capable of reacting with said chemically reactive substance to produce an insoluble color precipitate;

c) contacting said chemical component with said reagent to produce said soluble reaction product; and

d) contacting said substrate with said soluble reaction product so as to produce said insoluble color precipitate.

89. In a method for the multiple analysis of genetic material which comprises:

a) denaturing said genetic material;

b) fixing said genetic material to a substrate:

c) hybridizing with more than one probe wherein each of said probes is chemically labeled either directly or indirectly;

d) contacting each of said probes with a chemical component which attaches itself to said chemical label moiety and is effective on contact with a chromogen to produce an insoluble color precipitate; and

e) contacting each of said chemical components with a chromogen to produce an insoluble color precipitate, wherein each insoluble color precipitate from each probe can be distinguished.

90. A method in accordance with item 89 wherein each of said insoluble color precipitates from each of said probes is distinguished by forming

differently colored insoluble color precipitates or by forming said insoluble color precipitates sequentially.

91. A method for the analysis or detection of a selected biological material such as DNA, protein or a polypeptide, which comprises:

a) fixing said biological material to a substrate;

b) detecting said biological material with a chemically labeled probe;

c) contacting said chemically labeled probe with the first chemical component which attaches itself to said chemical label moiety of said probe and is effective on contact with a reagent to produce a reaction product that is capable of interacting with a second chemical component so as to convert said second chemical component to a form that is capable of interacting with a third chemical component to form a colored or visible product;

d) contacting said first chemical component with said reagent to produce said reaction product; and

e) contacting said reaction product with said second chemical component and said third chemical component to produce said colored or visible product.

The following examples are illustrative of the practices of this invention:

EXAMPLE I

Preparation of DNA

DNA of high molecular weight was isolated from HeLa Cells ($5X10^8$ cells) by a modification of the method of Blin and Stafford (Nuc. Acid Res. 3, 2303-2308 (1976). Briefly, a volume equal to the original sample volume of lysing solution (0.1M NaCl/0.05M Tris-JCl, pH 7.5/0.01M EDTA/0.5% SDS) was added and the cells were incubated at $37^oC$ for 10 minutes. Proteinase K (100 ug per ml of lysing solution) was then added and the lysed cells were incubated overnight at $37^oC$. The solution was then deproteinized by repeated extractions with phenol/chloroform (1:1) and the supernatant was dialyzed against 50mM Tris-HCl pH 8.0/10mM EDTA/10mM NaCl overnight at $4^oC$. Nucleic acids were precipitated by the addition of 2.5 volumes of 95% ethanol plus 0.1 volume 3M Na Acetate, incubated at $-70^oC$ for 30 minutes and collected by centrifugation at 11,000X g for 30 minutes. The pellet was resuspended in 10 ml 10mM

0124124

Tris-HCl pH 7.5/1mM EDTA. RNA was digested by the addition of 50 ug/ml of heat-treated RNase (RNase A, Sigma), followed by incubation at 37°C for 45 minutes, followed by phenol/chloroform. DNA was pelleted by ethanol precipitation followed by centrifugation and redissolved in sterile 0.5mM Tris-HCl at a concentration of 0.2 mg/ml.

Labeling DNA with Biotin

A pBR322 clone containing a 4.4 Kb PST I fragment of the human beta globin gene and flanking sequences was used as the probe. This probe was briefly nicked with DNase I and then "tailed" with terminal transferase in the presence of biotinylated-dUTP, or malto-triose-dUTP.

Restrictions, Electrophoresis and Transfer to Membranes

10-25 ug of genomic DNA was digested with a 4-fold excess of restriction endonuclease Bam HI under recommeded conditions. Digested samples were separated on 1% agarose gels in Tris-Acetate Buffer (pH 6.5). The DNA was then transferred to nitrocellulose (S+S, BA85) as described in Southern, E.M., JMB 98, 503-517 (1975). This "Southern Blot" was baked in a vacuum oven at 70°C for 2 hours.

Hybridization:

The baked nitrocellulose was wet in 2X SSC and then immersed in blocking solution (5X Denhardt's solution/5 X SSC/45% formamide/50mM phosphate buffer pH 6.5/1% glycine/0.1% SDS/1 mg/ml sonicated, denatured salmon sperm DNA) and incubated at 42° for at least 1 hour. The blot was then transferred to hybridization solution (1 X Denhardts solutions/5 X SSC/45% formamide/20mM phosphate

buffer, pH 6.5/0.1% Dextran Sulfate/1 mg/ml sonicated denatured salmon sperm DNA) containing from 20 ng to 1 ug of biotinylated- or malto-triose-labeled probe. The blot was incubated at 42°C overnight, and then washed twice in 2 X SSC, 0.1% SDS at room temperature (15 minutes each), then washed twice (15 minutes each) in the same solution at 68°C, then finally washed twice (for 15 minutes each) in 2 X SSC with no SDS.

Detection

Detection was performed on the Southern Blot as follows: the filter at this point contains biotinylated or malto-triose labeled DNA hybridized to the non-labeled genomic DNA on the blot. This filter is blocked by immersion in PBS containing 2% bovine serum albumin (BSA) and 0.1% X-100 and incubations at 37°C for 1/2 hours. Detections of biotinylated DNA is accomplished by the additions of a mixture of biotinylated horseradish peroxidase and streptavidin in 1% BSA (in 1 X PBS) directly to the blot. The minimum amount necessary is 0.018 $ml/cm^2$. This step can be performed in a sealed bag or in a plastic petri dish. After 30 minutes incubation at 37°C, the excess complex is washed off the filter as follows: 3 times, 5 minutes each in high salt buffer (0.5M NaCl/10mM phosphate buffer pH 6.5/0.1% BSA/0.05% Tween 20) and then twice, 5 minutes each in 2 X SSC/0.1% BSA/0.05% Tween 20.

Wherever biotinylated DNA was hybridized, horseradish peroxidase is now bound. This enzyme can be localized by the addition of a color-developing substrate which precipitates at the site of reaction. One such substrate is DAB (diamino benzidine). 5 mg of DAB is dissolved in 10 ml 10mM Tris pH 7.5. 200 ul of 1% cobalt chloride is added to the DAB solution, mixed, and left on ice for 10 minutes in the dark. Just prior to use, 70 ul of 30%

$H_2O_2$ are added to the DAB/cobalt solution. This substrate is pipetted onto the surface of the blot. Color appears in 10 seconds - 10 minutes depending on the amount of DNA on the filter.

Sensitivity

Experiments with cloned DNA, E. coli genomic DNA, and human genomic DNA have shown that detection with this system reveals clear signal with as little as 1 picogram of DNA. Attemtps to increase the sensitivity even further are now in progress. This sensitivity is sufficient to detect a single copy gene in human DNA. Since we can see single copy bands, even with as little as 1 ug of restricted genomic human DNA, the validity of this approach for pre- and post-natal genetic screening is clear.

Other Experiments

Our data show that this technique is applicable to other types of blot as well (Northern and Western ) as well as dot blots, plaque lifts and colony hybridization by adjusting hybridization times and probe concentrations. The detection aspect remains unchanged throughout the entire spectrum of techniques.

Other probes have been tested as well, also with success. In addition to terminal transferase, biotinylated dUTP can be incorporated into probe DNA by nick translation (Rigby, P.W. et al., JMB 113, 237 (1977)). Single-stranded probes, generated from M13 clones with the Klenow enzyme and biotinylated nucleotides have also been used successfully.

0124124

EXAMPLE II

DETECTION OF EPSTEIN-BARR DNA ON SOUTHERN BLOTS

Materials and Methods

Cloned cosmid and plasmid DNA carrying portions of the Epstein-Barr virus (EBV) genome were prepared according to the standard protocols. The plasmid clone (pDK14) contains the reiterated Bam V region of EBV in pBR322. Two cosmid clones, both in MUA3, were used; one (CNT126) contains the EcoRl AS fragment. Both of the cosmid clones contains DNA which is complementary to the Bam HI V region. All DNA samples were digested with Bam HI and electrophoresed on a 1% 1.5mm agarose gel in Tris-Acetate buffer, vs a lambda-Hind III digest as a size marker. After electrophoresis, the gel was stained with ethidium bromide and photographed under UV light. DNA was depurinated in situ for 15 minutes in 0.25N HCl, denatured for 2 X 15 minutes in 0.5M NaOH, 1.5M NaCl, and neutralized for 2 X 15 minutes in 0.5M Tris-Cl (pH 7.5), 1.5M NaCl. After soaking briefly in 20 X SSC, DNA was transferred to nitrocellulose as described by Southern. The filter was baked in vacuo for 2 hours at $80^oC$.

Plasmid (pDK14) DNA was nicked translated with $^{32}$p-dATP (Amersham) and biotinylate-dUTP. The probe was purified from unincorporated nucleotides by gel filtration on Sephadex G50, precipititated in ethanol, washed with 70% ethanol, dried and dissolved in water. The extent of substitution with bio-dUTP was 16.4% (of all avaiable T residues) and the specific activity of the DNA was 3000 cpm/ng. Probe size was between 200 and 700 base pairs in length.

The nitrocellulose blots were prehybridized at 42°C for 2 hours in sealed bags with 50% deionized formamide, 5 X SSC, 10% Dextran Sulfate, 10 X Denhardts solution, and 18 ug/ml deproteinized, sonicated salmon sperm DNA. Hybridization was carried out in 30% formamide, 25mM Na phosphate (pH 7.0), 10 X Denhardts solution, 10% Dextran Sulfate, 18 ug/ml salmon sperm DNA carrier, and 500 ng/ml of denatured pDK14 probe. The filter was incubated at 42°C for 20 hours, washed in 2 X SSC for 2 X 30 minutes at 37°C, and in 2 X SSC for 2 X 30 minutes at room temperature. The filter was then air dried and autoradiographed with screen at -70°C on Kodak XR-2 film.

Detection was performed as follows: the filters were wet in 1 X PBS, and incubated for 30 minutes, at 37°C in blocking solution (2% acid-treated BSA, 1 X PBS and 0.1% Trition X-100). A complex was formed between biotinylated horseradish peroxidase (HRP) and streptavidin (SA) by mixing in 1 X PBS with 1% BSA and incubating for 10 minutes at room temperature. Two ml of this complex was then spread over the blocked filter (DNA side up), and incubated at 37°C for 30 minutes. The filter was then washed 3 X 5 minutes in the high salt wash (0.5M NaCl, 10mM Na phosphate (pH 6.5), 0.1% BSA, and 0.5% Tween), and 2 X 5 minutes in a 2 X SSC wash containing 0.1% BSA and 0.0% Tween. 5 mg of the substrate (diaminobenzidine; DAB) was dissolved in 10 ml of 10mM Tris-Cl (pH8.0); 5 ul of 30% $H_2O_2$ was added, and the mixture was immediately applied to the filter. A visible product was formed in less than 5 minutes.

EXAMPLE III

Colony Hybridization - Preliminary Procedures

This example is illustrative of the practices of this invention to colony hybridization. The procedures folowed in this example are:

1. Clinical samples of *N. gonococcus* are grown on nitrocellulose over L. Agar at 37°C.

2. Remove the nitrocellulose paper from the petri dish and let air dry briefly.

3. Put the nitrocellulose paper on a clean petri dish and treat with lysozyme (1 mg/ml in 10mM Tris-Cl, pH 8.0,; 1mM EDTA) for 15 minutes at room temperature. Drop the lysozyme solution on the surface of the colonies.

4. Lay the nitrocellulose paper on two pieces of Whatman #3 paper saturated with 0.25M EDTA, pH 8.0, for 5 minutes.

5. Put nitrocellulose paper on Buchner funnel with a piece of Whatman 1 filter as support and apply suction for 1-2 minutes to remove excess liquid and stabilize colony.

6. Lay on filter paper which is saturated with:

    a. 0.5M NaOH for 5 minutes, then suction dry on Buchner funnel.

0124124

b. 0.5M Tris-Cl, pH 7.5, for 5 minutes, then suction dry on Buchner funnel

c. 0.5M Tris-Cl, 1.5M NaCl for 5 minutes, then suction dry on Buchner funnel

7. Treat with pronase (1 mg/ml in 10mM Tris-Cl, pH 7.5, self-digested) for 5 minutes. Add pronase dropwise to the surface of the colonies.

8. Lay the nitrocellulose paper on a funnel with Whatman paper as support and rinse with chloroform 5 times (1 minutes treatment each time).

9. Immerse in 3% $H_2O_2$ for 5 minutes to inactive endogenous HRP activity.

10. Rinse the nitrocellulose paper with 0.3N NaCl in a petri dish.

11. Air dry at room temperature or 37°C for 5-10 minutes.

12. Bake for 2 hours at 70°C in vacuo.

13. This paper can be stored in a sealed plastic bag at room temperature for several weeks.

14. Prehybridize with 6 X SSC + 1 X Denhardt solution + 50 ug/ml of heat-denatured calf thymus DNA at 65°C for 1-3 hours.

15. Scrape excess cell-debris from nitrocellulose paper.

0124124

16. Hybridize at 65°C with 6 X SSC, 1 X Denhardt solution with heat-denatured probe at 65°C overnight. (DNA probe for detection of Amp[r] is prepared from E. coli strain E. coli carrying a Col EI Tn 3 plasmid) and labeled with biotin by nick-translation).

17. Wash 3 times with 2 X SSC, 0.5% SDS at 65°C for approximately 1 hour each time.

## Detection of Hybridized Chemically Labeled Probe

1. Wash 2 times with 10mM Tris-Cl, pH 7.5, for 10 minutes.

2. React with HRP-SA complex for 30 minutes to 1 hour at 37°C in petri dish.

3. Wash with the following high salt solution (3 times, 25 ml each time in a beaker with gentle agitation):

| FINAL | STOCK | FOR 100 ML |
|---|---|---|
| 0.5M NaCl | 5M | 10ml |
| 10mM $NaPO_4$ p.H 5.6 | 1M | 1ml |
| 0.1% acidified BSA | 10% | 1ml |
| 0.5 ml/1 Tween 20 | | 50ul |

4. Low salt wash (2 times, 25 ml each time, with gentle agitation):

0124124

| FINAL | STOCK | FOR 100 ml |
|---|---|---|
| 2 X SSC | 20X | 10 ml |
| 0.1 acidified BSA | 10% | 1 ml |
| 0.5 ml/1 Tween 20 | 100% | 50 ul |

5. React with DAB (freshly prepared diaminobenzidine

| | 10 ml | 5 ml | 2 ml | 1 ml |
|---|---|---|---|---|
| DAB | 5 mg | 2.5 mg | 1 mg | 0.5 mg |
| $CoCl_2$ 1% | 200 ul | 100 ul | 40 ul | 20 ul |

Wait about 10 minutes on ice in dark by wrapping in aluminum foil.

| | | | | |
|---|---|---|---|---|
| $H_2O_2$ 30% | 160 ul | 80 ul | 32 ul | 16 ul |

Note: 1. High $H_2O_2$ concentration is used here is order to decrease the paper (nonspecific) background.

2. Drop on gently, wait for reaction. It takes 1-10 minutes at room temperature.

6. Soak in 10mM Tris-Cl, pH 7.5, to stop reaction.

## Confirmation of a Negative Result

1. Place the nitrocellulose paper in an ethidium bromide solution for 1 minute.

2. Place the nitrocellulose paper under ultra-violet light.

3. To keep the nitrocellulose paper, seal in a plastic bag with Tris-buffer at room temperature.

0124124

EXAMPLE IV

RNA DOT BLOTS

| | |
|---|---|
| Nitrocellulose paper | |
| Glyoxal | 6.0M |
| SSC | 20X |
| Na phosphate (pH 7.1) | 1.0M |

Preparation of Nitrocellulose Paper

1. Wet nitrocellulose paper in water. Soak for 10 minutes in 20 X SSC. Air dry.

2. Glyoxylation of RNA: RNA up to 20 ug is incubated at 50°C for 60 minutes in 5mM Na phosphatase, pH 7.1, 1M Glyoxal. Quench to 20°C in ice.

3. Serial dilutions of RNA are made in 5mM Na phosphatase, pH 7.1, 1M Glyoxal and 1 ul aliquots spotted on the nitrocellulose paper. Air dry.

4. Bake at 80°C in vacuo for 2-4 hours.

5. Rinse strips in large volume of 2 X SSC at 65°C for 60 minutes each, 3 times, 20 minute each, in 2 X SSC at room temperature.

   Air dry. Store in a sealed bag up to 4-8 weeks.

## RNA Dot Blot Hybridization

Preparation of biotinylated nick-translated or terminal transferased DNA probe:

Ribosomal DNA clone in bacteriophage lambda was biotinylated either by nick-translation or labeled terminally with biotinylated nucleotides using terminal transferase using established protocols. Extent of incorporation was monitored by using $^{32}$p-dATP.

The probe was purified free of unincorporated nucleotides and enzymes and kept frozen at -20°C.

1. Prehybridization of nitrocellulose strips with RNA blots was carried out at 42°/C for 8-20 hours in 50% Formamide/5 X SSC/50mM Na phosphate, pH 6.5/250 ug/ml denatured salmon sperm DNA/0.02% BSA/PVP/Ficoll.

2. Hybridization was carried out at 42°C for 20 hours in 4 parts prehybridization buffer/1 part 50% Dextran Sulfate/100 ng/ml denatured probe.

3. Rinse filters at room temperature 4 times in 2 X SSC + 0.1% SDS for 5 minutes each/50°C 2 times in 0.1 X SSC + 0.1% DS for 15 minutes each.
Air dry.

## Detection

1. Wet strips in 1 X PBS.

2. Block strips in 2% acidified BSA/1 PBS/0.1% Trition X-100 at 37°C for 30 minutes.
   Pour off blocking solution.

3. Apply streptavidin-biotinylated horseradish peroxidase in 1% BSA/1 X PBS/0.018 ml/$cm^2$.

   Incubate at 37°C for 30-60 minutes.

4. Rinse strips 3 times, 5 minutes each, in 0.5M NaCl/0.1% BSA/0.05% Tween 20/10mM potassium phosphate, pH 6.5, and 2 times, 5 minutes each, in 2 X SSC/0.1% BSA/0.05% Tween 20.

5. Apply substrate in the dark.

## EXAMPLE V

## DETECTION RNA-DNA HYBRIDIZATION NORTHERN BLOTS

### Glyoxylation of RNA

| Final Conc. | | |
|---|---|---|
| E. coli ribosomal RNA (8.0 mg/ml) | 3.7 ul | |
| 6M Glyoxal | 2.7 ul | 1.0M |
| Dimethylsuloxide | 8.0 ul | 50% |
| $NaPO_4$, pH 7.1, 0.1M | 1.6 ul | 10mM |

Incubate at 50°C for 60 minutes. Cool to 20°C. Add 4 ul of sterile loading after buffer (50% Glycerol/10M $NaPO_4$, pH 7.0/0.4% Bromophenol Blue).

Gel Electrophoresis

Immediately apply to 1.4% agarose gel in 10mM $NaPO_4$, the sample driven into gel at 20 volts, the electrophoresis carried out at 100 volts for 2 hours with buffer recirculating.

Staining

Do not stain the gel if it is to be used for transfer.

Stain gel in 0.05% ug/ml ethidium bromide in $H_2$ for 20 minutes in the dark. Wash free of ethidium bromide.

Northern Blot Transfer

1. RNA from the gel is tranferred to nitrocellulose paper in 20 X SSC at room temperature for 20-24 hours.

2. Dry nitrocellulose paper under heat lamp for 15 minutes.

3. Bake in vaccum oven at 80°C for 2 hours.

   Store in sealed bag.

4. Prehybridize at 42°C for 8-20 hours in 50% Formamide/5 X DNA/5 X SSC.

5. Hybridize at 42°C for 20 hours in 50% Formamide/10% Dextran Sulfate/0.1% SDS/5 X SSC/1-2 X Denhardt solution/100-200 ug/ml denatured carrier DNA denatured probe.

0124124

6. Rinse 2 times, 10 minutes each, in 2 X SSC + 0.1% SDS 2 times, 10 minutes each, in 0.1 X SSC + 0.1% SDS at 50°C. Air dry.

Detection

1. Wet the filter in 1 X PBS.

2. Block the filter in 2% BSA/0.1% Triton X-100/1 X PBS at 37°/C for 30 minutes.

3. Remove blocking solution. Do not wash.

4. Apply streptavidin-biotinylated horseradish peroxidase in 1% BSA + 1 X PBS at 37°C for 30-60 minutes.

5. Rinse filter 3 times, 5 minutes each in 0.5M NaCl/0.1% BSA/BSA/0.5% Tween/10mM $KPO_4$, pH 6.5, and 2 times, 5 minutes each, in 2 X SSC/0.1% BSA/0.05%/Tween 20.

0124124

EXAMPLE VI

Detection of DNA by Southern Blot Technique on Nitrocellulose and Nylon Membranes

In the experiments Herpes genomic DNA (HSV-1) strain F was digested with Bam H1 and subjected to electrophorsis on 1% agarose gel in Tris-borate buffer (pH 8.3). The amount of DNA per lane subjected to electrophoresis varied from 1 ug to 100 pg. Upon completion of the fractionation of the DNA material by electrophoresis, the gel was soaked in 2.5M NaCl and 0.5M NaOH for 30 minutes and then transferred to 3M Na acetate (pH 5.5) for 30 minutes. The transfer from gel to the membranes, a nylon membrane (Pall Biodyne A membrane) and a nitrocellulose sheet or membrane was carried out in accordance with the technique described by Southern. The substrates or filters or membranes were dried and baked in a vaccum oven at 70°C. for 2 hours, blocked in 5 X Denhardts, 5 X SSC, 50% deionized formamide, 50mM Na phosphate (pH 6.8), 1% glycine, 1 mg/ml sonicated, denatured calf thymus and DNA and 0.1% SDS for 2 hours at 42°C. and then transferred to the hybridization solution. The hybridization solution contained 1 X Denhardts, 2 X SSC, 50% deionized formamide, 20 mM Na phosphate (pH 6.8), 0.1% SDS, 10% dextran sulfate, 1 mg/ml sonicated denatured calf thymus DNA and 800 mg/5ml of nick translated HSV- which was 48% substituted with biotinylated-dUPT. Hybridization was performed overnight at 42°C. and the filters or membranes washed in 5 mM $NaH_2PO_4$, 1 mM EDTA, 0.2% SDS, 2 X 15 min. at room temperature, then 2 X 15 min. at 68°C, and finally 1 X 15 minute at room temperature. The membranes or filters were then blocked and detected with streptavidin-biotinylated horseradish peroxidase.

0124124

It was noted that the nylon membrane, the Pall Biodyne A membrane, had lower background, better band resolution and approximately a 2-fold increase in sensitivity relative to the nitrocellulose substrate or membrane.

EXAMPLE VII

Detection of DNA Employing Colony Hybridization

*E. Coli* plasmid DNA containing Tn3 (*amp*$^r$) gene was isolated and labeled by means of terminal transferase and biotinylated dUTP. Clinical cultures of *amp*$^r$ and *amp*$^s$ of *Neisseria gonorroeae* (GC) were grown on a nylon transfer membrane, Pall biodyne transfer membrane, (1-2 ug BNNG 8225) and processed by the colony hybridization technique described hereinabove. The biotinylated DNA probe, after removing the substrates on a G-50 column was used for hybridization with the colonies. Those amp$^r$ and amp$^s$ GC gave the excepted results by detection with streptavidin-biotinylated horseradish peroxidase and a chromogen. The amp$^r$ colonies gave a brown color or response and the sensitive colonies were colorless or gave a colorless response.

EXAMPLE VIII

In Situ Detection of Herpes Simplex Virus I and/or II

Vero cells were placed on a glass slide and were then infected with Herpes Simplex Virus I and/or II (HSV I and/or II) at a multiplicity of infection of 20 to 1 for 14 hours of infection. The vero cells were washed and fixed in Carnoy's B fixative for 5 minutes at room temperature and then air dried. (Carnoy's B fixative comprises 60% ethanol, 30% chloroform and 10% acetic acid).

Three clones carrying portions of the HSV I and/or II genome in pBR322 were prepared at the Bam HI region according to the standard protocol. The inserts were 5.2 Kb, 1.9 Kb and 13.5 Kb. The 3 clones were then labeled by nick translation in the presence of biotinylated dUTP. The extent of substitution with biotinylated dUTP was about 25% of all available T residues. Probe sizes were between 200 and 1150 base pairs in length.

A carrier mix comprising 60ug/ml of probe and 10mg of carrier DNA, which was sonicated calf thymus DNA, was prepared. The hybridization mix was then prepared and comprised 12ng/microliter probe, 2mg/ml carrier DNA, 10% dextran sulfate, 2 X SSC and 50% formamide. The hybridization mix was mixed well and then 20 microliters were added to the slide. A coverslip was applied over the slide and then the slide was sealed with tape.

The probe was then denatured by the following procedure: A stand was placed in a water bath so that the surface of the stand was above the water level. A petri dish with wet filter paper on its surface was placed on the surface of the stand. The slide was then placed on the surface of the filter paper. The water was heated -- such that the temperature immediately above the surface of the water was 80°C. The petri dish which contains the slide, was removed after 10 minutes.

Hybridization was carried out as follows:
The petri dish with slide in place, was removed from the water bath and incubated at 37°C for 60 minutes.

0124124

That portion of the probe which did not hybridized was removed as follows:
The tape on the coverslip was removed and the coverslip was removed by immersing the slide in 2 X SSC. The slide was then washed 5 times. The first wash was for 5 minutes at room temperature in 2 X SSC. The next 3 washes were for 5 minutes each at 65°C in 0.1X SSC. The fifth wash was for 5 minutes at room temperature in 2 X SSC. The slide was then immersed in 0.1% Triton X100 in phosphate buffer saline (PBS) for 2 minutes. The slide was rinsed in PBS for 5 minutes and then the excess liquid was shaken off.

Biotinylated horseradish peroxidase-streptavidin complex was added as follows:
The complex was added to a dilution buffer comprising of PBS and bovine serum albumin (BSA) with a resulting concentration of 2 microliters/0.5ml of the complex. About 100 microliters of such buffer was added to the slide. The slide was then incubated at 37°C for 30 minutes. The slide was then washed to remove that portion of the complex which did not complex with the biotinylated probe. This was carried out as follows:
The slide was washed for 2 minutes with 2 X SSC and then for 2 minutes with 0.1% Triton X100 and then for 5 minutes with PBS. The excess liquid was then shaken off.

The assay for peroxidase was carried out as follows:
A solution comprising 2.5mg of diaminobenzidine tetrahydrochloride and 5ml of PBS was prepared. 100 microliters of 1% $H_2O_2$ was added to such solution. 100 microliters of the solution was added to the slide. After about 5 minutes the reaction was stopped by dipping the slide in water. The slide was then observed

under a microscope and a rust brown color was observed indicating the presence of HSV I and/or HSV II.

EXAMPLE IX

In Situ Detection of Epstein Barr Virus

Marmoset cells persistantly infected with Epstein Barr Virus (EBV) strain B 95-8 were prepared. The infected marmoset cells contained about 200 copies of EBV genome per cell. The cells were applied to polylysine coated slides and were allowed to stick. The cells were then washed and fixed in Carnoy's B fixative for 5 minutes at room temperature and then air dried.

One clone carrying a portion of the EBV genome in pBR322 was prepared at the Bam V region according to the standard protocol. The insert was 3.071 Kb. The probe was then labeled by nick translation in the presence of biotinylated dUTP. The extent of substitution with biotinylated dUTP was about 50% of all available T residues. Probe sizes were between 200 and 2000 base pairs in length.

The protocol for the detection of EBV was carried out as described in Example VIII. The slide was then observed under a microscope and a rust brown color which indicates the presence of EBV was observed.

As will be apparent to those skilled in the art in the light of the foregoing disclosure, many substitutions, alterations and modifications are possible in the practices of this invention without departing from the spirit or scope thereof.

EXAMPLE X

Lambda DNA was restricted with Hind III and electrophoresed on a 1% Agarose gel in Tris-Borate-EDTA (pH 8.3). The gel was loaded so that each lane contained either 100ng, 10ng, 1ng, 100pg or 10pg of restricted DNA. When the gel had been run for 2 1/2 hours at 100ma., the electrophoresis was stopped. The gel was then immersed in 0.5M NaOH, 1.5M NaCL, for 30 minutes. The gel was then transferred to 0.5M Tris-HCl (pH 8.0), 1.5M NaCl, 3 changes for 15 minutes each. The DNA was then transferred to nitrocellulose with 20 X SSC using standard protocols. When the transfer was complete, the nitrocellulose blot was rinsed in 2 X SSC and baked in vacuo for 2 hours at 80$^{o}$C.

The nitrocellulose blot was soaked in 2 X SSC and incubated with 5 X SSC, 5 X Denharts solution, 0.1% SDS and 100ug/ml of sonicated denatured salmon sperm DNA for 2 hours at 65$^{o}$C. The nitrocellulose blot was then tranferred to a solution containing 5 X SSC, 1 X Denharts solutions, 0.1% SDS, 100ug/ml of sonicated denatured salmon sperm DNA and 200ng/ml of Lambda DNA nick translated with biotin-dUTP according to standard protocols for 17 hours at 65$^{o}$C. About 36% of the T's of the probe were replaced with biotin-dUTP.

Excess unhybridized probe was washed away by immersing the nitrocellulose blot in 2 X SSC, 0.1% SDS at 65$^{o}$C for 15 minutes. This was repeated 3 times. The blot was transferred to a solution of 2% bovine serum albumin, 1 X phosphate buffered saline, 0.05% Triton-X-100, for 30 minutes at 37$^{o}$C. This solution was pipetted off and the enzyme solution was added. This solution contained 1ug/ml of glucose oxidase which had

been coupled to biotin (using the biotin-C7-NHS ester at pH 8) at a molar ratio of 10 biotins to 1 enzyme. The biotinylated enzyme was complexed with streptavidin at a ratio of 4: 1 (wt:wt). This complex was incubated with the hybridized nitrocellulose blot at 37°C for 30 minutes.

Excess (unbound) complex was then washed away by 5 sequential rinses in 0.5m NaCl, 10ml Tris-HCl (pH 7.5) for 10 minutes each.

An image was then obtained from the nitrocellulose blot by placing it on a piece of Whatman 3MM blotter paper saturated with a solution of 1M KCl and 1M B-D-glucose. A piece of Pall Biodyne A membrane (previously dipped in a saturated solutions of soluble starch and dried) was then placed on both the nitrocellulose blot, which is on the Whatman paper. The entire sandwich was then incubated at 37°C for 1 hour. The layers of the sandwich were then carefully separated. All 3 layers had a clear image of the lambda bands down to the 41 pg band. The nitrocellulose blots were dried and stored in plastic bags.

EXAMPLE XI

The same procedure as Example X was utilized, but lipoxidase was used instead of glucose oxidase and linoleic acid replaced glucose. The same result as in Example X was observed.

As will be apparent to those skilled in the art in the light of the foregoing disclosure, many substitutions, alternations and modifications are possible in the practices of this invention without departing from the spirit or scope thereof.

0124124

WHAT IS CLAIMED IS:

1. In a method for the analysis of genetic material, such as DNA and/or RNA, wherein the genetic material is denatured, fixed to a substrate and hybridized with a probe, the improvement which comprises employing as said probe a chemically labeled probe, said chemically labeled probe having attached thereto a chemical component effective upon contact with a chromogen to produce an insoluble color precipitate.

2. A method in accordance with Claim 1 wherein said component is attached to said chemical label after said probe has been hybridized with said genetic material fixed to said substrate.

3. A kit useful for the analysis of genetic material comprising a chemically labeled nucleotide, an enzyme complex comprising an enzyme effective upon contact with a chromogen to produce an insoluble color precipitate, a chromogen effective contact with said enzyme of said enzyme complex to produce an insoluble color precipitate, and enzyme means for terminally labeling a single stranded polynucleotide probe with said chemically labeled nucleotide or for the incorporation of said chemically labeled nucleotide, such as by nick translation, to produce said probe.

4. In a method for the analysis of genetic material, such as DNA or RNA, wherein genetic material is denatured, fixed in situ or to a substrate and hybridized with a probe the improvement which comprises employing as said probe a chemically

labeled probe, said chemically labeled probe having attached thereto a chemical component effective upon contact or reaction with a chromogen to produce a colored or visible product or an insoluble color precipitate.

5. A method for the analysis of protein, protein-like or peptide material which comprises solubilizing said material, subjecting the resulting solubilizing material to fractionation, contacting the resulting fractionerial material with a complex, said complex comprising anted material with a complex said complex comprising an antibody for a selected component of said fractionated material, said complex having attached to the antibody thereof a first reactant material and fixing of said complex via said antibody to said select component, thereupon contacting the resulting complexed fractionated material with a chemical reagent comprising a second reactant material, said second reactant material being capable of complexing with or attaching to said first reactant material, said chemical reagent also comprising a third reactant material being effective upon contact or reaction with a chromogen to produce a colored or visible product or bringing said chromogen into contact with said chemical reagent to produce said colored or visible product or insoluble color precipitate, thereby eliciting or identifying the presence of said selected component of said fractionated material.

0124124

6. In a method for the analysis or detection of a selected biological material, such as DNA, protein or a polypeptide, wherein the biological material is fractionated, fixed to a substrate and detected by a probe which attaches itself to the selected biological material to be determined or improvement which comprises employing as said probe a probe which attaches itself to said selected biological material, said probe being provided with a chemical label moiety which is detectable by contact with a chemical component which attaches itself to said chemical label moiety of said probe and which is effective upon contact with a chromogen to produce a colored or visible product or insoluble color precipitate.

7. A kit for the analysis of genetic material comprising terminal transferase, a biotinylated or glycosylated nucleotide as a substrate for said terminal transferase an enzyme complex for attachment to said biotinylated of glycosylated nucleotide and a membrane or filter substrate material selected from nitrocellulose paper or nylon membrane.

8. A kit for the analysis of genetic material comprising:

A) a chemically labeled probe capable of hybridizing with at least a portion of said genetic material.

B) an enzyme complex attachable to said chemical label, said enzyme complex comprising an enzyme effective on contact with a chromogen to produce an insoluble color precipitate;

C) a chromogen effective on contact with said enzyme of said enzyme complex to produce an insoluble color precipitate.

9. A kit for the identification of proteins or peptides comprising:

A) a chemically labeled protein or peptide probe;

B) an enzyme complex attachable to said chemical label, said enzyme complex comprising an enzyme effective upon contact with a chromogen to produce an insoluble color precipitate;

C) a chromogen effective on contact with said enzyme of said enzyme complex to produce an insoluble color precipitate.

10. A method for the analysis or detection of a selected biological material such as DNA, protein or a polypeptide, which comprises:

a) fixing said biological material to a substrate;

b) detecting said biological material with a chemically labeled probe;

c) contacting said chemically labeled probe with a chemical component which attaches itself to said chemical label moiety of said probe and is effective on contact with a reagent to produce a reaction product that is capable of oxidizing soluble iodide to iodine;

0124124

d) contacting said chemical component with said reagent product that is capable of oxidizing iodide to iodine; and

e) contacting said reaction product with a soluble iodide and starch so as to produce a colored or visible product.

11. In a method for the multiple analysis of genetic material which comprises:

a) denaturing said genetic material;

b) fixing said genetic material to a substrate:

c) hybridizing with more than one probe wherein each of said probes is chemically labeled either directly or indirectly;

d) contacting each of said probes with a chemical component which attaches itself to said chemical label moiety and is effective on contact with a chromogen to produce an insoluble color precipitate; and

e) contacting each of said chemical components with a chromogen to produce an insoluble color precipitate, wherein each insoluble color precipitate from each probe can be distinguished.

12. A method for the analysis or detection of a selected biological material such as DNA, protein or a polypeptide, which comprises:

   a) fixing said biological material to a substrate;

   b) detecting said biological material with a chemically labeled probe;

   c) contacting said chemically labeled probe with the first chemical component which attaches itself to said chemical label moiety of said probe and is effective on contact with a reagent to produce a reaction product that is capable of interacting with a second chemical component so as to convert said second chemical component to a form that is capable of interacting with a third chemical component to form a colored or visible product;

   d) contacting said first chemical component with said reagent to produce said reaction product; and

   e) contacting said reaction product with said second chemical component and said third chemical component to produce said colored or visible product.